# EUROPÄISCHE PATENTSCHRIFT

(11) **EP 1 937 648 B1**
(45) Veröffentlichungstag und Bekanntmachung des Hinweises auf die Patenterteilung: **26.11.2014**
(21) Anmeldenummer: 06806017.7
(22) Anmeldetag: 04.10.2006
(51) Int. Cl.: C07D 231/56, A61K 31/416, A61P 3/10

(54) **DIACYLINDAZOL-DERIVATE ALS INHIBITOREN VON LIPASEN UND PHOSPHOLIPASEN**
DIACYL INDAZOL DERIVATIVES AS LIPASE AND PHOSPHOLIPASE INHIBITORS
DÉRIVÉS D'INDAZOLE DE DIACYLE CONSTITUANT DES INHIBITEURS DE LIPASES ET DE PHOSPHOLIPASES

(30) Priorität: 12.10.2005 DE 102005048897
(43) Veröffentlichungstag der Anmeldung: 02.07.2008
(73) Patentinhaber: SANOFI, 75008 Paris (FR)
(72) Erfinder: ZOLLER, Gerhard, 65926 Frankfurt am Main (DE); PETRY, Stefan, 65926 Frankfurt am Main (DE); MÜLLER, Günter, 65926 Frankfurt am Main (DE); HEUER, Hubert, 65926 Frankfurt am Main (DE); TENNAGELS, Norbert, 65926 Frankfurt am Main (DE)
(74) Vertreter: Essler, Frank
(86) Internationale Anmeldenummer: PCT/EP2006/009577
(87) Internationale Veröffentlichungsnummer: WO 2007/042178

(56) Entgegenhaltungen:
- EP-A- 1 164 421
- WO-A-03/035005
- WO-A-2004/035550
- WO-A-2004/037814
- WO-A-2004/093872
- WO-A-2005/073199

## Beschreibung

Die vorliegende Erfindung betrifft Diacylindazol-derivate der allgemeinen Formeln I oder II, deren pharmazeutisch anwendbare Salze und deren Verwendung als Arzneistoffe.

Strukturähnliche Verbindungen sind in der WO 2005/073199 beschrieben. Zum Stand der Technik zählt auch die WO2003/035005.

Aufgabe der vorliegenden Erfindung ist die Bereitstellung von alternativen Verbindungen, die eine Hemmung der hormonsensitiven Lipase oder der endothelialen Lipase bewirken.

Gegenstand der Erfindung sind Diacylindazol-derivate der allgemeinen Formeln I oder II, 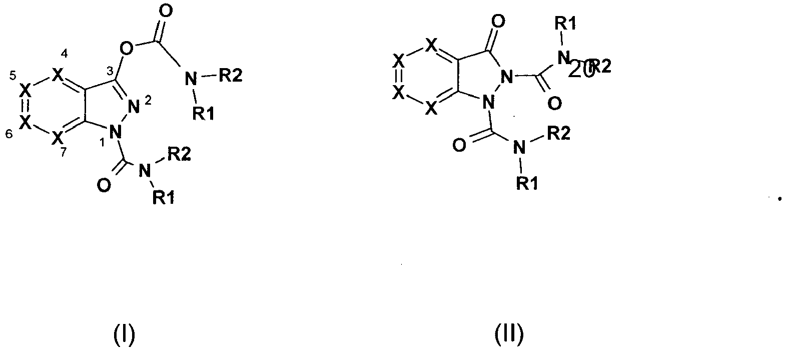 wobei bedeuten:
- X: gleich oder verschieden =C(-R)- oder =N-, wobei höchstens ein X für =N- steht;
- R: gleich oder verschieden Wasserstoff, Halogen, Hydroxy, (C₁-C₆)-Alkyl, Trifluormethyl, (C₁-C₃)-Alkyloxy oder Amino;
- R1: gleich oder verschieden (C₆-C₁₀)-Alkyl oder Y-Phenyl, das durch Halogen, (C₁-C₆)-Alkyl, (C₁-C₃)-Alkyloxy oder Trifluormethyl substituiert sein kann;
- Y: CH₂;
- R2: Wasserstoff; oder
- R1 und R2: zusammen mit dem sie tragenden Stickstoffatom ein monocyclisches, gesättigtes 5- bis 6-gliedriges Ringsystem bilden, deren einzelne Glieder durch ein bis drei Atome oder Atomgruppen aus der Reihe -CHR4-, - NR4-, -O-, -S- ersetzt sein können, mit der Maßgabe, dass zwei Einheiten aus der Reihe -O-, -S- nicht benachbart sein dürfen;
- R4: (C₁-C₆)-Alkyl, Trifluormethyl oder Phenyl bedeuten;
die tautomeren Formen der Verbindungen sowie deren physiologisch verträgliche Salze.

Besonders bevorzugt sind auch die Verbindungen der Formeln I oder II, worin
- NR1 R2: für ein monocyclisches gesättigtes 5- bis 6-gliedriges Ringsystem steht, das in 4 Position ein Atom oder Atomglied aus der Reihe -CHR4-, - CR4R5-, -(C=R4)-, -NR4-, -O-, -S- enthält.

Insbesondere ganz besonders bevorzugt sind die Verbindungen der Formeln I oder II, worin
- X: gleich oder verschieden =C(-R)- oder =N-, wobei höchstens ein X für =N- steht;
- R: gleich oder verschieden Wasserstoff, F, Cl, Hydroxy, Methyl, Trifluormethyl oder Amino;
- R1: gleich oder verschieden (C₆-C₁₀)-Alkyl oder Y-Phenyl, welches durch Methyl substituiert sein kann;
- Y: CH₂;
- R2: Wasserstoff; oder
- R1 und R2: zusammen mit dem sie tragenden Stickstoffatom ein monocyclisches, gesättigtes 5- bis 6-gliedriges Ringsystem bilden, deren einzelne Glieder durch ein bis drei Atome oder Atomgruppen aus der Reihe -CHR4-, - NR4-, -O-, -S- ersetzt sein können, mit der Maßgabe, dass zwei Einheiten aus der Reihe -O-, -S- nicht benachbart sein dürfen;
- R4: Methyl, Trifluormethyl oder Phenyl, welches durch Methyl oder Cl ein oder zweifach substituiert sein kann;
bedeuten;
die tautomeren Formen der Verbindungen sowie deren physiologisch verträgliche Salze.

Insbesondere besonders bevorzugt sind auch die Verbindungen der Formeln I oder II, worin
NR1 R2 für Piperidin steht, das in 4 Position das Atomglied -CHR4- enthält. Bevorzugt sind weiterhin Verbindungen der Formeln I oder II, worin
- X: gleich oder verschieden =C(-R)- bedeutet.

Bevorzugt sind auch die Verbindungen der Formeln I oder II, worin
- X: in Position 4, 5 und 6 für gleich oder verschieden =C(-R)-, in Position 7 für =N- steht.

Ferner sind bevorzugt Verbindungen der Formeln I oder II, worin
- X: in Position 4, 5 und 7 für =C(-R)- mit R = Wasserstoff steht, in Position 6 R nicht für Wasserstoff steht.

Die Erfindung bezieht sich auf Verbindungen der Formeln I oder II, in Form ihrer Salze, Racemate, racemischen Mischungen und reinen Enantiomere sowie auf ihre Diastereomere und Mischungen davon.

Die Alkylreste in den Substituenten R, R1, R2, R3, R4, R5, R6 und R7 können sowohl geradkettig wie verzweigt sein. Halogen steht für Fluor, Chlor, Brom oder lod, besonders für Fluor oder Chlor.
Haloalkyl sind Alkylreste, bei denen ein, mehrere oder alle Wasserstoffatome durch Halogen, bevorzugt Fluor ersetzt sind.

Unter einem Arylrest wird ein Phenyl oder Naphthylrest verstanden.

Unter einem Cycloalkylrest wird ein einen oder mehrere Ringe enthaltendes Ringssystem, welches gesättigt oder partiell ungesättigt (mit einer oder zwei Doppelbindungen) vorliegt, verstanden, das ausschließlich aus Kohlenstoffatomen aufgebaut ist, wie z.B. Cyclopropyl, Cyclopentyl, Cyclopentenyl, Cyclohexyl oder Adamantyl.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Äpfel-, Methansulfon-, Bernstein-, p-Toluolsulfon- und Weinsäure. Geeignete pharmazeutisch verträgliche basische Salze sind Ammoniumsalze, Alkalimetallsalze (wie Natrium- und Kaliumsalze) und Erdalkalisalze (wie Magnesium- und Calciumsalze) und Salze von Trometamol (2-Amino-2-hydroxymethyl-1,3-propandiol), Diethanolamin, Lysin oder Ethylendiamin.

Salze mit einem nicht pharmazeutisch verträglichen Anion, wie zum Beispiel Trifluoracetat, gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Der hier verwendete Begriff "physiologisch funktionelles Derivat" bezeichnet jedes physiologisch verträgliche Derivat einer erfindungsgemäßen Verbindung der Formeln I oder II, z.B. einen Ester, der bei Verabreichung an einen Säuger, wie z.B. den Menschen, in der Lage ist, (direkt oder indirekt) Verbindungen der Formeln I oder II oder einen aktiven Metaboliten hiervon zu bilden.

Zu den physiologisch funktionellen Derivaten zählen auch Prodrugs der erfindungsgemäßen Verbindungen, wie zum Beispiel in H. Okada et al., Chem. Pharm. Bull. 1994, 42, 57-61 beschrieben. Solche Prodrugs können in vivo zu einer erfindungsgemäßen Verbindung metabolisiert werden. Diese Prodrugs können selbst wirksam sein oder nicht.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formeln I oder II "auf Verbindung(en) der Formeln I oder II, wie vorstehend beschrieben, sowie ihre Salze, Solvate und physiologisch funktionelle Derivate wie hierin beschrieben.

### Verwendung

Die erfindungsgemäßen Verbindungen der allgemeinen Formeln I oder II besitzen eine überraschende hemmende Wirkung an der Hormon Sensitiven Lipase, HSL, einem allosterischen Enzym in Adipozyten, das durch Insulin gehemmt wird und für den Abbau von Fetten in Fettzellen und damit für die Überführung von Fettbestandteilen in die Blutbahn verantwortlich ist. Eine Hemmung dieses Enzyms entspricht also einer insulinähnlichen Wirkung der erfindungsgemäßen Verbindungen, die letztlich zu einer Verminderung von freien Fettsäuren im Blut und von Blutzucker führt. Sie können also eingesetzt werden bei Entgleisungen des Stoffwechsels wie zum Beispiel beim nicht insulinabhängigen Diabetes Mellitus, beim diabetischen Syndrom und bei direkter Schädigung des Pankreas.
Außerdem können die erfindungsgemäßen Verbindungen der allgemeinen Formeln I oder II eine hemmenden Wirkung auf die endotheliale Lipase (EL) besitzen. Das antiatheroskleotisch wirksame HDL ist das bevorzugte Substrat für EL. Eine Senkung des HDL-Spiegels führt zur Progression von Atherosklerose und ihrer Folgeerkrankungen wie Metabolisches Syndrom und Koronare Herzerkrankungen. Eine Hemmung der EL sollte somit zur Vorbeugung von atherosklerotischen Erkrankungen führen.
Weiterhin wurde gefunden, dass die hemmende Wirkung der erfindungsgemäßen Verbindungen der allgemeinen Formeln I oder II selektiv gegenüber anderen Lipasen ist.

Besonders geeignet sind solche Verbindungen zur Behandlung und/oder Prävention von
1. - Störungen des Fettsäurestoffwechsels und Glucoseverwertungsstörungen
2. Störungen der Insulin-Sensitivität von Muskel-, Fett- und Leberzellen (Insulin-Resistenz) - Metabolisches Syndrom
3. Diabetes meltitus, insbesondere Typ 2 Diabetes einschließlich der Verhinderung der damit verbundenen Folgeerkrankungen.
   Besondere Aspekte sind dabei die
   - Hyperglykämie,
   - Verbesserung der Insulinresistenz,
   - Verbesserung der Glucose-Toleranz,
   - Schutz der β-Zellen der Bauchspeicheldrüse
   - Verhinderung makro- und mikrovaskulärer Erkrankungen
4. Dyslipidämien und deren Folgen, wie z.B. Atherosklerose, koronare Herzkrankheit, zerebrovaskuläre Erkrankungen etc, insbesondere solche (aber nicht beschränkt auf), die durch einen oder mehrerer folgender Faktoren charakterisiert sind:
   - hohe Plasma-Triglycerid-, hohe postprandiale Plasma-Triglycerid-Konzentrationen
   - niedrige HDL-Cholesterin Konzentration
   - niedrige ApoA Lipoprotein-Konzentrationen
   - hohe LDL-Cholesterin Konzentrationen
   - kleine dichte LDL-Cholesterin Partikel
   - hohe ApoB Lipoprotein-Konzentrationen
5. Verschiedenen andere Zuständen, die mit dem Metabolischen Syndrom assoziert sein können, sind wie:
   - Adipositas (Fettsucht), einschließlich abdominale Adipositas
   - Thrombosen, Stadien von Hyperkoagulabilität und Thromboseneigung (arteriell und venös)
   - Hoher Blutdruck
   - Herzinsuffizienz, wie z.B. (aber nicht beschränkt auf) bei Zustand nach Myokardinfarkt, hypertensive Herzerkrankung oder Kardiomyopathie
6. weitere Krankheiten oder Zustände bei welchen zum Beispiel entzündliche Reaktionen oder die Zelldifferenzierung eine Rolle spielt sind:
   - Atherosklerose wie z.B. (aber nicht beschränkt auf) Koronarsklerose einschl. Angina pectoris oder Herzinfarkt, Hirnschlag
   - Vaskuläre Restenose oder Reverschluß
   - Chronisch entzündliche Darmerkrankungen, wie z.B. Morbus Crohn und Colitis ulcerosa
   - Pankreatitis
   - Andere entzündliche Zustände
   - Retinopathie
   - Fettzell-Tumore (adipose cell tumors)
   - Fettzell-Karzinome, wie z.B. Liposarkome
   - solide Tumoren und Neoplasien, wie z.B. (aber nicht beschränkt auf) Karzinome des Magen-Darm Traktes, der Leber, der Gallenwege und des Pankreas, endokrine Tumore, Karzinome der Lunge, der Niere und harnableitenden Organe, des Genitaltraktes, Prostata-Karzinome etc.
   - akute und chronische myeloprolifeative Erkrankungen und Lymphome
   - Angiogenese
   - Neurodegenerative Erkrankungen
   - Alzheimersche Krankheit
   - Multiple Sklerose
   - Morbus Parkinson
   - Erythemato-squamöse Dermatosen, wie z.B. Psoriasis (Schuppenflechte)
   - Akne vulgaris
   - Andere Hautkrankheiten und dermatologische Zustände, die durch PPAR moduliert werden
   - Ekzeme und Neurodermitis
   - Dermatitiden, wie z.B. seborrhoische Dermatitis oder Lichtdermatitis
   - Keratitis und Keratosen, wie z.B. seborrhoische Keratosen, senile Keratosen, aktinische Keratose, photo-induzierte Keratosen oder Keratosis follicularis
   - Keloide und Keloid-Prophylaxe
   - Warzen, einschließlich Kondylomata oder Kondylomata acuminata
   - Human papilloma viral (HPV) Infektionen, wie z.B. venerische Papillomata, virale Warzen, wie z.B. Molluscum contagiosum, Leukoplakie
   - Papulöse Dermatosen, wie z.B. Lichen planus
   - Hautkrebs, wie z.B. Basalzellkarzinome, Melanome oder kutane T-Zell Lymphome
   - Lokalisierte, benigne epidermale Tumore, wie z.B. Keratoderma, epidermale Naevi
   - Frostbeulen
   - Hoher Blutdruck
   - Syndrom X
   - Syndrom der polyzystischen Ovarien (PCOS)
   - Asthma
   - Osteoarthritis
   - Lupus erythematodes (LE) oder entzündliche rheumatische Erkrankungen, wie z.B. Rheumatoide Arthritis
   - Vaskulitis
   - Auszehrung (Kachexie)
   - Gicht
   - Ischämie/Reperfusions Syndrom
   - Akutes respiratorisches Distress Syndrom (ARDS) ("Schocklunge")

### Galenik

Die Menge einer erfindungsgemäßen Verbindung, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg bis 50 mg) pro Tag pro Kilogramm Körpergewicht, z.B. 3-10 mg/kg/Tag.

Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 0.05 bis 1000 mg, typischerweise von 0,5 bis 600 mg enthalten. Zur Therapie der oben genannten Zustände können die Verbindungen gemäß Formeln I oder II selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer erfindungsgemäßer Verbindungen. Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindungen gemäß Formeln I oder II abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinylacetatphthalat, Hydroxypropylmethylcellulosephthalat und anionische Polymere von Methacrylsäure und Methacrylsäuremethylester.

Geeignete pharmazeutische Zubereitungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formeln I oder II enthalten; als Pulver oder Granulate; als Lösung oder Suspension in einer wässrigen oder nicht-wässrigen Flüssigkeit; oder als eine Öl-in-Wasser- oder Wasser-in Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mitteln in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formeln I oder II mit einem Geschmacksstoff enthalten, üblicherweise Saccharose und Gummi arabicum oder Tragant, und Pastillen, welche die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wässrige Zubereitungen von Verbindungen gemäß Formeln I oder II, die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formeln I oder II mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglycole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wässrigen Lösung, gelöst und/oder dispergiert in einem Haftmittel oder dispergiert in einem Polymer. Eine geeignete Wirkstoff-Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder lontophorese freigesetzt werden.

Die Verbindungen der Formeln I und II zeichnen sich durch günstige Wirkungen auf Stoffwechselstörungen aus. Sie beeinflussen den Fett- und Zuckerstoffwechsel positiv, sie senken insbesondere den Triglyceridspiegel und sind zur Prävention und Behandlung von Typ II Diabetes und Arteriosklerose sowie deren vielfältigen Folgeerkrankungen geeignet.

### Kombinationen mit anderen Medikamenten

Die erfindungsgemäßen Verbindungen können alleine oder in Kombination mit einem oder mehreren weiteren pharmakologischen Wirkstoffen verabreicht werden. Insbesondere können die erfindungsgemäßen Verbindungen mit Wirkstoffen, die eine ähnliche pharmakologische Wirkung wie sie selbst aufweisen, verabreicht werden. Beispielsweise können sie in Kombination mit Wirkstoffen, die eine günstige Wirkungen auf Stoffwechselstörungen bzw. häufig damit assoziierte Erkrankungen haben, verabreicht werden. Beispiele für solche Medikamente sind
1. blutzuckersenkende Medikamente, Antidiabetika,
2. Wirkstoffe zur Behandlung von Dyslipidämien,
3. antiatherosklerotische Medikamente,
4. Mittel gegen Obesitas,
5. entzündungshemmende Wirkstoffe
6. Wirkstoffe zur Behandlung von malignen Tumoren
7. antithrombotische Wirkstoffe
8. Wirkstoffe zur Behandlung von Bluthochdruck
9. Wirkstoffe zur Behandlung von Herzinsuffizienz und
10. Wirkstoffe zur Behandlung und/oder Prävention von durch Diabetes verursachten bzw. mit Diabetes assoziierten Komplikationen.
11. Wirkstoffe zur Behandlung von neurodegenerativen Krankheiten
12. Wirkstoffe zur Behandlung von Krankheiten des Zentralen Nervensystems
13. Wirkstoffe zur Behandlung von Drogen-, Nikotin- und Alkoholabhängigkeit
14. Schmerzmittel

Sie lassen sich mit den erfindungsgemäßen Verbindungen der Formel I oder II insbesondere zur synergistischen Wirkungsverbesserung kombinieren. Die Verabreichung der Wirkstoffkombination kann entweder durch getrennte Verabreichung der Wirkstoffe an den Patienten oder in Form von Kombinationsprodukten, bei denen mehrere Wirkstoffe in einer pharmazeutischen Zubereitung vorhanden sind, erfolgen.

Als weitere Wirkstoffe für die Kombinationspräparate sind insbesondere geeignet: Alle Antidiabetika, die in der Roten Liste 2006, Kapitel 12 genannt sind; alle Abmagerungsmittel/Appetitzügler, die in der Roten Liste 2006, Kapitel 1 genannt sind; alle Lipidsenker, die in der Roten Liste 2006, Kapitel 58 genannt sind. Sie können mit der erfindungsgemäßen Verbindung der Formel I oder II insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen. Die meisten der nachfolgend aufgeführten Wirkstoffe sind in USP Dictionary of USAN and International Drug Names, US Pharmacopeia, Rockville 2001, offenbart.

Antidiabetika umfassen Insulin und Insulinderivate, wie z.B. Lantus^{®} (siehe www.lantus.com) oder HMR 1964 oder solche, wie sie in WO2005005477 (Novo Nordisk) beschrieben sind, schnell wirkende Insuline (siehe US 6,221,633), inhalierbare Insuline, wie z. B. Exubera^{®} oder orale Insuline, wie z. B. IN-105 (Nobex) oder Oral-lyn™ (Generex Biotechnology), GLP-1-Derivate wie z.B. Exenatide, Liraglutide oder diejenigen die in WO 98/08871 oder WO2005027978 von Novo Nordisk A/S, in WO 01/04156 von Zealand oder in WO 00/34331 von Beaufour-Ipsen offenbart wurden, Pramlintide Acetat (Symlin; Amylin Pharmaceuticals), sowie oral wirksame hypoglykämische Wirkstoffe.

Die Wirkstoffe umfassen vorzugsweise
Sulfonylharnstoffe,
Biguanide,
Meglitinide,
Oxadiazolidindione,
Thiazolidindione,
Glukosidase-Inhibitoren,
Hemmstoffe der Glykogen Phosphorylase,
Glukagon-Antagonisten,
Glukokinase Aktivatoren,
Inhibitoren der Fructose-1,6-bisphosphatase,
Modulatoren des Glukosetransporters-4 (GLUT4),
Inhibitoren der Glutamin-Fructose-6-Phosphat-Amidotransferase (GFAT), GLP-1-Agonisten,
Kaliumkanalöffner, wie z.B. diejenigen, die in WO 97/26265 und WO 99/03861 von Novo Nordisk A/S offenbart wurden,
Inhibitoren der Dipeptidylpeptidase-IV (DPP-IV),
Insulin-Sensitizer,
Inhibitoren von Leberenzymen, die an der Stimulation der Glukoneogenese und/oder Glykogenolyse beteiligt sind,
Modulatoren der Glukoseaufnahme, des Glukosetransports und der Glukoserückresorption,
Hemmstoffe der 11 β-HSD1,
Inhibitoren der Protein-Tyrosin-Phosphatase-1B (PTP1 B),
Modulatoren des natrium-abhängigen Glukosetransporters 1 oder 2 (SGLT1, SGLT2), den Fettstoffwechsel verändernde Verbindungen wie antihyperlipidämische Wirkstoffe und antilipidämische Wirkstoffe,
Verbindungen, die die Nahrungsmitteleinnahme verringern,
Verbindungen, die die Thermogenese erhöhen,
PPAR- und RXR-Modulatoren und
Wirkstoffe, die auf den ATP-abhängigen Kaliumkanal der Betazellen wirken.

Bei einer Ausführungsform der Erfindung wird die Verbindung der Formel I oder II in Kombination mit einem HMGCoA-Reduktase Inhibitor, wie Simvastatin, Fluvastatin, Pravastatin, Lovastatin, Atorvastatin, Cerivastatin, Rosuvastatin oder L-659699, verabreicht.

Bei einer Ausführungsform der Erfindung wird die Verbindung der Formel I oder II in Kombination mit einem Cholesterin resorptions inhibitor, wie z.B. Ezetimibe, Tiqueside, Pamaqueside, FM-VP4 (sitostanol/campesterol ascorbyl phosphat; Forbes Medi-Tech, WO2005042692), MD-0727 (Microbia Inc., WO2005021497) oder mit Verbindungen, wie in WO2002066464 (Kotobuki Pharmaceutical Co. Ltd.), WO2005062824 (Merck & Co.) oder WO2005061451 und WO2005061452 (AstraZeneca AB) beschrieben, verabreicht.

Bei einer Ausführungsform der Erfindung wird die Verbindung der Formel I oder II in Kombination mit einem PPAR gamma Agonisten, wie z.B. Rosiglitazon, Pioglitazon, JTT-501, GI 262570, R-483 oder CS-011 (Rivoglitazon), verabreicht.

Bei einer Ausführungsform der Erfindung wird die Verbindung der Formel I oder II in Kombination mit einem PPAR alpha Agonisten, wie z.B. GW9578, GW-590735, K-111, LY-674, KRP-101 oder DRF-10945, verabreicht.

Bei einer Ausführungsform der Erfindung wird die Verbindung der Formel I oder II in Kombination mit einem gemischten PPAR alpha/gamma Agonisten, wie z.B. Muraglitazar, Tesaglitazar, Naveglitazar, LY-510929, ONO-5129, E-3030 oder wie in WO00/64888, WO00/64876, WO03/020269, WO2004075891, WO2004076402, WO2004075815, WO2004076447, WO2004076428, WO2004076401, WO2004076426, WO2004076427, WO2006018118, WO2006018115, and WO2006018116 oder in J.P. Berger et al., TRENDS in Pharmacological Sciences 28(5), 244-251, 2005 beschrieben, verabreicht.

Bei einer Ausführungsform der Erfindung wird die Verbindung der Formel I oder II in Kombination mit einem PPAR delta Agonisten, wie z.B. GW-501516, oder wie in WO2005097762, WO2005097786, WO2005097763, WO2006029699 beschrieben verabreicht.

Bei einer Ausführungsform der Erfindung wird die Verbindung der Formel I oder II in Kombination mit Metaglidasen oder mit MBX-2044 oder anderen partiellen PPAR gamma Agonisten/Antagonisten verabreicht.

Bei einer Ausführungsform der Erfindung wird die Verbindung der Formel I oder II in Kombination mit einem Fibrat, wie z.B. Fenofibrat, Clofibrat oder Bezafibrat, verabreicht.

Bei einer Ausführungsform der Erfindung wird die Verbindung der Formel I oder II in Kombination mit einem MTP-Inhibitor, wie z.B. Implitapide, BMS-201038, R-103757 oder solchen wie in WO2005085226 beschrieben, verabreicht.

Bei einer Ausführungsform der Erfindung wird die Verbindung der Formel I oder II in Kombination mit einem CETP-Inhibitor, wie z.B. Torcetrapib oder JTT-705, verabreicht.

Bei einer Ausführungsform der Erfindung wird die Verbindung der Formel I oder II in Kombination mit einem Gallensäureresorptionsinhibitor (siehe z.B. US 6,245,744, US 6,221,897 oder WO00/61568), wie z.B. HMR 1741 oder solchen wie in DE 10 2005 033099.1 und DE 10 2005 033100.9 beschrieben, verabreicht.

Bei einer Ausführungsform der Erfindung wird die Verbindung der Formel I oder II in Kombination mit einem polymeren Gallensäureadsorber, wie z.B. Cholestyramin oder Colesevelam, verabreicht.

Bei einer Ausführungsform der Erfindung wird die Verbindung der Formel I oder II in Kombination mit einem LDL-Rezeptorinducer (siehe US 6,342,512), wie z.B. HMR1171, HMR1586 oder solchen wie in WO2005097738 beschrieben, verabreicht.

Bei einer Ausführungsform wird die Verbindung der Formel I oder II in Kombination mit Omacor® (Omega-3-Fettsäuren; hochkonzentrierte Ethylester der Eicosapentaensäure und der Docosahexaensäure) verabreicht.

Bei einer Ausführungsform der Erfindung wird die Verbindung der Formel I oder II in Kombination mit einem ACAT-Inhibitor, wie z.B. Avasimibe, verabreicht.

Bei einer Ausführungsform der Erfindung wird die Verbindung der Formel I oder II in Kombination mit einem Antioxidans, wie z.B. OPC-14117, Probucol, Tocopherol, Ascorbinsäure, β-Caroten oder Selen, verabreicht.

Bei einer Ausführungsform der Erfindung wird die Verbindung der Formel I oder II in Kombination mit einem Vitamin, wie z. B. Vitamin B6 oder Vitamin B12, verabreicht.

Bei einer Ausführungsform der Erfindung wird die Verbindung der Formel I oder II in Kombination mit einem Lipoprotein-Lipase Modulator, wie z.B. Ibrolipim (NO-1886), verabreicht.

Bei einer Ausführungsform der Erfindung wird die Verbindung der Formel I oder II in Kombination mit einem ATP-Citrat-Lyase Inhibitor, wie z.B. SB-204990, verabreicht.

Bei einer Ausführungsform der Erfindung wird die Verbindung der Formel I oder II in Kombination mit einem Squalen Synthetase Inhibitor, wie z.B. BMS-188494 oder wie in WO2005077907 beschrieben, verabreicht.

Bei einer Ausführungsform der Erfindung wird die Verbindung der Formel I oder II in Kombination mit einem Lipoprotein(a) Antagonist, wie z.B. Gemcabene (Cl-1027), verabreicht.

Bei einer Ausführungsform der Erfindung wird die Verbindung der Formel I oder II in Kombination mit einem HM74A Rezeptor Agonisten, wie z.B. Nicotinsäure, verabreicht.

Bei einer Ausführungsform der Erfindung wird die Verbindung der Formel I oder II in Kombination mit einem Lipase Inhibitor, wie z.B. Orlistat oder Cetilistat (ATL-962), verabreicht.

Bei einer Ausführungsform der Erfindung wird die Verbindung der Formel I oder II in Kombination mit Insulin verabreicht.

Bei einer Ausführungsform der Erfindung wird die Verbindung der Formel I oder II in Kombination mit einem Sulfonylharnstoff, wie z.B. Tolbutamid, Glibenclamid, Glipizid oder Glimepirid, verabreicht.

Bei einer Ausführungsform der Erfindung wird die Verbindung der Formel I oder II in Kombination mit einem Biguanid, wie z.B. Metformin, verabreicht.

Bei wieder einer Ausführungsform der Erfindung wird die Verbindung der Formel I oder II in Kombination mit einem Meglitinid, wie z.B. Repaglinide oder Nateglinid, verabreicht.

Bei einer Ausführungsform der Erfindung wird die Verbindung der Formel I oder II in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]phenyl]methyl]-2,4-thiazolidindion, verabreicht.

Bei einer Ausführungsform der Erfindung wird die Verbindung der Formel I oder II in Kombination mit einem α-Glukosidase-Inhibitor, wie z.B. Miglitol oder Acarbose, verabreicht.

Bei einer Ausführungsform der Erfindung wird die Verbindung der Formel I oder II in Kombination mit einem Wirkstoff verabreicht, der auf den ATP-abhängigen Kaliumkanal der Betazellen wirkt, wie z.B. Tolbutamid, Glibenclamid, Glipizid, Glimepirid oder Repaglinid.

Bei einer Ausführungsform der Erfindung wird die Verbindung der Formel I oder II in Kombination mit mehr als einer der vorstehend genannten Verbindungen, z.B. in Kombination mit einem Sulfonylharnstoff und Metformin, einem Sulfonylharnstoff und Acarbose, Repaglinid und Metformin, Insulin und einem Sulfonylharnstoff, Insulin und Metformin, Insulin und Troglitazon, Insulin und Lovastatin, etc., verabreicht.

Bei einer Ausführungsform der Erfindung wird die Verbindung der Formel I oder II in Kombination mit einem Hemmstoff der Glykogen Phosphorylase, wie z.B. PSN-357 oder FR-258900 oder solchen wie in WO2003084922, WO2004007455, WO2005073229-31 oder WO2005067932 beschrieben, verabreicht.

Bei einer Ausführungsform der Erfindung wird die Verbindung der Formel I oder II in Kombination mit Glukagon-Rezeptor-Antagonisten, wie z.B. A-770077, NNC-25-2504 oder wie in WO2004100875 oder WO2005065680 beschrieben, verabreicht.

Bei einer Ausführungsform der Erfindung wird die Verbindung der Formel I oder II in Kombination mit Aktivatoren der Glukokinase, wie z. B. RO-4389620, LY-2121260 (WO2004063179), PSN-105, PSN-110, GKA-50oder solchen wie sie z. B. von Prosidion in WO2004072031, WO2004072066, WO 05103021 oder WO 06016178, von Roche in WO 00058293, WO 00183465, WO 00183478, WO 00185706, WO 00185707, WO 01044216, GB 02385328, WO 02008209, WO 02014312, WO 0246173, WO 0248106, DE 10259786, WO 03095438, US 04067939 oder WO 04052869, von Novo Nordisk in EP 1532980, WO 03055482, WO 04002481, WO 05049019, WO 05066145 oder WO 05123132, von Merck/Banyu in WO 03080585, WO03097824, WO 04081001, WO 05063738 oder WO 05090332, von Eli Lilly in WO 04063194, oder von Astra Zeneca in WO 01020327, WO 03000262, WO 03000267, WO 03015774, WO 04045614, WO 04046139, WO 05044801, WO 05054200, WO 05054233, WO 05056530, WO 05080359, WO 05080360 oder WO 05121110 beschrieben sind, verabreicht.

Bei einer Ausführungsform der Erfindung wird die Verbindung der Formel I oder II in Kombination mit einem Inhibitor der Glukoneogenese, wie z. B. FR-225654, verabreicht.

Bei einer Ausführungsform der Erfindung wird die Verbindung der Formel I oder II in Kombination mit Inhibitoren der Fructose-1,6-bisphosphatase (FBPase), wie z.B. CS-917, verabreicht.

Bei einer Ausführungsform der Erfindung wird die Verbindung der Formel I oder II in Kombination mit Modulatoren des Glukosetransporters-4 (GLUT4), wie z.B. KST-48 (D.-O. Lee et al.: Arzneim.-Forsch. Drug Res. 54 (12), 835 (2004)), verabreicht.

Bei einer Ausführungsform der Erfindung wird die Verbindung der Formel I oder II in Kombination mit Inhibitoren der Glutamin-Fructose-6-Phosphat-Amidotransferase (GFAT), wie sie z. B. in WO2004101528 beschrieben sind, verabreicht.

Bei einer Ausführungsform der Erfindung wird die Verbindung der Formel I oder II in Kombination mit Inhibitoren der Dipeptidylpeptidase-IV (DPP-IV), wie z. B. Vildagliptin (LAF-237), Sitagliptin (MK-0431), Saxagliptin ((BMS-477118), GSK-823093, PSN-9301, SYR-322, SYR-619, TA-6666, TS-021, GRC-8200, GW-825964X oder wie sie in WO2003074500, WO2003106456, WO200450658, WO2005058901, WO2005012312, WO2005/012308, PCT/EP2005/007821, PCT/EP2005/008005, PCT/EP2005/008002, PCT/EP2005/008004, PCT/EP2005/008283, DE 10 2005 012874.2 oder DE 10 2005 012873.4 beschrieben sind, verabreicht.

Bei einer Ausführungsform der Erfindung wird die Verbindung der Formel I oder II in Kombination mit Hemmstoffen der 11-beta-Hydroxysteroid-Dehydrogenase-1 (11ß-HSD1), wie z. B. BVT-2733 oder solche, wie sie z. B. in WO200190090-94, WO200343999, WO2004112782, WO200344000, WO200344009, WO2004112779, WO2004113310, WO2004103980, WO2004112784, WO2003065983, WO2003104207, WO2003104208, WO2004106294, WO2004011410, WO2004033427, WO2004041264, WO2004037251, WO2004056744, WO2004065351, WO2004089367, WO2004089380, WO2004089470-71, WO2004089896, WO2005016877 oder WO2005097759 beschrieben sind, verabreicht.
Bei einer Ausführungsform der Erfindung wird die Verbindung der Formel I oder II in Kombination mit Inhibitoren der Protein-Tyrosin-Phosphatase-1B (PTP1 B), wie sie z. B. in WO200119830-31, WO200117516, WO2004506446, WO2005012295, PCT/EP2005/005311, PCT/EP2005/005321, PCT/EP2005/007151, PCT/EP2005/01294 oder DE 10 2004 060542.4 beschrieben sind, verabreicht.

Bei einer Ausführungsform der Erfindung wird die Verbindung der Formel I oder II in Kombination mit Modulatoren des natrium-abhängigen Glukosetransporters 1 oder 2 (SGLT1, SGLT2), wie z.B. KGA-2727, T-1095 und SGL-0010 oder wie sie z. B. in WO2004007517, WO200452903, WO200452902, WO2005121161, WO2005085237, JP2004359630 oder von A. L. Handlon in Expert Opin. Ther. Patents (2005) 15(11), 1531-1540 beschrieben sind, verabreicht.

Bei einer Ausführungsform der Erfindung wird die Verbindung der Formel I oder II in Kombination mit Inhibitoren der hormon-sensitiven Lipase (HSL), wie z. B. in WO01/17981, WO01/66531, WO2004035550, WO2005073199 oder WO03/051842 beschrieben, verabreicht.

Bei einer Ausführungsform der Erfindung wird die Verbindung der Formel I oder II in Kombination mit Hemmstoffen der Acetyl-CoA Carboxylase (ACC) wie z. B. solchen wie in WO199946262, WO200372197, WO2003072197 oder WO2005044814 beschrieben, verabreicht.

Bei einer Ausführungsform der Erfindung wird die Verbindung der Formel I oder II in Kombination mit einem Inhibitor der Phosphoenolpyruvatcarboxykinase (PEPCK), wie z.B. solchen, wie in WO2004074288 beschrieben, verabreicht.

Bei einer Ausführungsform der Erfindung wird die Verbindung der Formel I oder II in Kombination mit einem Inhibitor der Glykogen Synthase Kinase-3 beta (GSK-3 beta), wie z. B. in US2005222220, WO2004046117, WO2005085230, WO2005111018, WO2003078403, WO2004022544, WO2003106410, WO2005058908, US2005038023, WO2005009997, US2005026984, WO2005000836, WO2004106343, EP1460075, WO2004014910, WO2003076442, WO2005087727 oder WO2004046117 beschrieben, verabreicht.

Bei einer Ausführungsform der Erfindung wird die Verbindung der Formel I oder II in Kombination mit einem Inhibitor der Protein Kinase C beta (PKC beta), wie z. B. Ruboxistaurin, verabreicht.

Bei einer Ausführungsform der Erfindung wird die Verbindung der Formel I oder II in Kombination mit einem Endothelin-A-Rezeptor Antagonisten, wie z. B. Avosentan (SPP-301), verabreicht.

Bei einer Ausführungsform der Erfindung wird die Verbindung der Formel I oder II in Kombination mit Inhibitoren der "I-kappaB Kinase" (IKK Inhibitoren), wie sie z. B. in WO2001000610 WO2001030774, WO2004022553 oder WO2005097129 beschrieben sind, verabreicht.

Bei einer Ausführungsform der Erfindung wird die Verbindung der Formel I oder II in Kombination mit Modulatoren des Glukocorticoidrezeptors, wie sie z. B. in WO2005090336 beschrieben sind, verabreicht.

Bei einer weiteren Ausführungsform der Erfindung wird die Verbindung der Formel I oder II in Kombination mit CART-Modulatoren (siehe "Cocaine-amphetamine-regulated transcript influences energy metabolism, anxiety and gastric emptying in mice" Asakawa, A. et al.: Hormone and Metabolic Research (2001), 33(9), 554-558); NPY-Antagonisten wie z.B. Naphthalin-1-sulfonsäure-{4-[(4-amino-quinazolin-2-ylamino)-methyl]-cyclohexylmethyl}-amid Hydrochlorid (CGP 71683A);
Peptid YY 3-36 (PYY3-36) oder analoge Verbindungen, wie z. B. CJC-1682 (PYY3-36 konjugiert mit humanem Serum Albumin über Cys34), CJC-1643 (Derivat des PYY3-36, welches sich in vivo an Serum Albumin konjugiert) oder solche, wie sie in WO2005080424 beschrieben sind;
Cannabinoid Rezeptor 1 Antagonisten, wie z.B. Rimonabant, SR147778 oder solche wie sie in z. B. EP 0656354, WO 00/15609, WO 02/076949, WO2005080345, WO2005080328, WO2005080343, WO2005075450, WO2005080357, WO200170700, WO2003026647-48, WO200302776, WO2003040107, WO2003007887, WO2003027069, US6,509,367, WO200132663, WO2003086288, WO2003087037, WO2004048317, WO2004058145, WO2003084930, WO2003084943, WO2004058744, WO2004013120, WO2004029204, WO2004035566, WO2004058249, WO2004058255, WO2004058727, WO2004069838, US20040214837, US20040214855, US20040214856, WO20040962 WO2004096763, WO2004096794, WO2005000809, WO2004099157, US20040266845, WO2004110453, WO2004108728, WO2004000817, WO2005000820, US20050009870, WO200500974, WO2004111033-34, WO200411038-39, WO2005016286, WO2005007111, WO2005007628, US20050054679, WO2005027837, WO2005028456, WO2005063761-62, WO2005061509 oder WO2005077897 beschrieben sind;
MC4-Agonisten (z.B. 1-Amino-1,2,3,4-tetrahydro-naphthalin-2-carbonsäure [2-(3a-benzyl-2-methyl-3-oxo-2,3,3a,4,6,7-hexahydro-pyrazolo[4,3-c]pyridin-5-yl)-1-(4-chloro-phenyl)-2-oxo-ethyl]-amid; (WO 01/91752)) oder LB53280, LB53279, LB53278 oder THIQ, MB243, RY764, CHIR-785, PT-141 oder solche wie sie in WO2005060985, WO2005009950, WO2004087159, WO2004078717, WO2004078716, WO2004024720, US20050124652, WO2005051391, WO2004112793, WOUS20050222014, US20050176728, US20050164914, US20050124636, US20050130988, US20040167201, WO2004005324, WO2004037797, WO2005042516, WO2005040109, WO2005030797, US20040224901, WO200501921, WO200509184, WO2005000339, EP1460069, WO2005047253, WO2005047251, EP1538159, WO2004072076, WO2004072077 oder WO2006024390 beschrieben sind;
Orexin-RezeptorAntagonisten (z.B. 1-(2-Methyl-benzoxazol-6-yl)-3-[1,5]naphthyridin-4-yl-harnstoff Hydrochlorid (SB-334867-A) oder solche, wie sie z. B. in WO200196302, WO200185693, WO2004085403 oder WO2005075458 beschrieben sind);
Histamin H3 Rezeptor Agonisten (z. B. 3-Cyclohexyl-1-(4,4-dimethyl-1,4,6,7-tetrahydro-imidazo[4,5-c]pyridin-5-yl)-propan-1-on Oxalsäuresalz (WO 00/63208) oder solche, wie sie in WO200064884, WO2005082893 beschrieben sind);
CRF-Antagonisten (z.B. [2-Methyl-9-(2,4,6-trimethyl-phenyl)-9H-1,3,9-triaza-fluoren-4-yl]-dipropyl-amin (WO 00/66585));
CRF BP-Antagonisten (z.B. Urocortin);
Urocortin-Agonisten;
β3-Agonisten (wie z.B. 1-(4-Chloro-3-methanesulfonylmethyl-phenyl)-2-[2-(2,3-dimethyl-1H-indol-6-yloxy)-ethylamino]-ethanol Hydrochlorid (WO 01/83451));
MSH (Melanocyt-stimulierendes Hormon)-Agonisten;
MCH (melanin-konzentrierendes Hormon) Rezeptor Antagonisten (wie z. B. NBI-845, A-761, A-665798, A-798, ATC-0175, T-226296, T-71, GW-803430 oder solche Verbindungen, wie sie in WO2003/15769, WO2005085200, WO2005019240, WO2004011438, WO2004012648, WO2003015769, WO2004072025, WO2005070898, WO2005070925, WO2006018280, WO2006018279, WO2004039780, WO2003033476, WO2002006245, WO2002002744, WO2003004027 oder FR2868780 beschrieben sind);
CCK-A Agonisten (wie z.B. {2-[4-(4-Chloro-2,5-dimethoxy-phenyl)-5-(2-cyclohexyl-ethyl)-thiazol-2-ylcarbamoyl]-5,7-dimethyl-indol-1-yl}-essigsäure Trifluoressigsäuresalz (WO 99/15525), SR-146131 (WO 0244150) oder SSR-125180);
Serotonin-Wiederaufnahme-Inhibitoren (z.B. Dexfenfluramine);
gemischte Serotonin- und noradrenerge Verbindungen (z.B. WO 00/71549); 5-HT-Rezeptor Agonisten z.B. 1-(3-Ethyl-benzofuran-7-yl)-piperazin Oxalsäuresalz (WO 01/09111);
5-HT2C Rezeptor Agonisten (wie z.B. APD-356, BVT-933 oder solche, wie sie in WO200077010, WO20077001-02, WO2005019180, WO2003064423, WO200242304 oder WO2005082859 beschrieben sind);
5-HT6 Rezeptor Antagonisten, wie sie z.B. in WO2005058858 beschrieben sind; Bombesin-Rezeptor Agonisten (BRS-3 Agonisten);
Galanin-Rezeptor Antagonisten;
Wachstumshormon (z.B. humanes Wachstumshormon oder AOD-9604); Wachstumshormon freisetzende Verbindungen (6-Benzyloxy-1-(2-diisopropylamino-ethylcarbamoyl)-3,4-dihydro-1H-isochinolin-2-carbonsäuretertiärbutylester (WO 01/85695));
Growth Hormone Secretagogue Receptor Antagonisten (Ghrelin Antagonisten) wie z. B. A-778193 oder solche, wie sie in WO2005030734 beschrieben sind; TRH-Agonisten (siehe z.B. EP 0 462 884);
entkoppelnde Protein 2- oder 3-Modulatoren;
Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881);
DA-Agonisten (Bromocriptin oder Doprexin);
Lipase/Amylase-Inhibitoren (wie sie z.B. in WO 00/40569 beschrieben sind); Inhibitoren der Diacylglycerol O-Acyltransferasen (DGATs) wie z. B. in US2004/0224997, WO2004094618, WO200058491, WO2005044250, WO2005072740, JP2005206492 oder WO2005013907 beschrieben;
Inhibitoren der Fettsäuresynthase (FAS) wie z.B. C75 oder solche, wie in WO2004005277 beschrieben;
Oxyntomodulin;
Oleoyl-Estron

oder Agonisten des Schilddrüsenhormonrezeptors (thyroid hormone receptor agonists) wie z. B: KB-2115 oder solche, wie in WO20058279 WO200172692, WO200194293, WO2003084915, WO2004018421 oder WO2005092316 beschrieben, verabreicht.

Bei einer Ausführungsform der Erfindung ist der weitere Wirkstoff Leptin; siehe z.B. "Perspectives in the therapeutic use of leptin", Salvador, Javier; Gomez-Ambrosi, Javier; Fruhbeck, Gema, Expert Opinion on Pharmacotherapy (2001), 2(10), 1615-1622.

Bei einer Ausführungsform der Erfindung ist der weitere Wirkstoff Dexamphetamin oder Amphetamin.

Bei einer Ausführungsform der Erfindung ist der weitere Wirkstoff Fenfluramin oder Dexfenfluramin.

Bei noch einer Ausführungsform der Erfindung ist der weitere Wirkstoff Sibutramin.

Bei einer Ausführungsform der Erfindung ist der weitere Wirkstoff Mazindol oder Phentermin.

Bei einer Ausführungsform der Erfindung wird die Verbindung der Formel I oder II in Kombination mit Ballaststoffen, vorzugsweise unlöslichen Ballaststoffen (siehe z.B. Carob/ Caromax^{®} (Zunft H J; et al., Carob pulp preparation for treatment of hypercholesterolemia, ADVANCES IN THERAPY (2001 Sep-Oct), 18(5), 230-6). Caromax ist ein Carob enthaltendes Produkt der Fa. Nutrinova, Nutrition Specialties & Food Ingredients GmbH, Industriepark Höchst, 65926 Frankfurt / Main) verabreicht. Die Kombination mit Caromax^{®} kann in einer Zubereitung erfolgen oder durch getrennte Gabe von Verbindungen der Formel I oder II und Caromax^{®}. Caromax^{®} kann dabei auch in Form von Lebensmitteln, wie z.B. in Backwaren oder Müsliriegeln, verabreicht werden.

Bei einer Ausführungsform der Erfindung wird die Verbindung der Formel I oder II in Kombination mit PDE Inhibitoren (Phosphodiesterase), wie sie z. B. in WO2003/077949 oder WO2005012485 beschrieben sind, verabreicht.

Bei einer Ausführungsform der Erfindung wird die Verbindung der Formel I oder II in Kombination mit NAR-1 (Nicotinic Acid Receptor) Agonisten, wie sie z. B. in WO2004094429 beschrieben sind, verabreicht.

Bei einer Ausführungsform der Erfindung wird die Verbindung der Formel I oder II in Kombination mit CB2 (Cannabinoid Receptor) Agonisten, wie sie z. B. in US2005/143448 beschrieben sind, verabreicht.

Bei einer Ausführungsform der Erfindung wird die Verbindung der Formel I oder II in Kombination mit Histamin-1-Agonisten, wie sie z. B. in WO2005101979 beschrieben sind, verabreicht.

Bei einer Ausführungsform der Erfindung wird die Verbindung der Formel I oder II in Kombination mit Bupropion, wie in WO2006017504 beschrieben, verabreicht.

Bei einer Ausführungsform der Erfindung wird die Verbindung der Formel I oder II in Kombination mit Opioid Antagonisten, wie sie z. B. in WO2005107806 oder WO2004094429 beschrieben sind, verabreicht.

Bei einer Ausführungsform der Erfindung wird die Verbindung der Formel I oder II in Kombination mit Neutralen Endopeptidase Inhibitoren , wie sie z. B. in WO200202513, WO2002/06492, WO 2002040008, WO2002040022 oder WO2002047670 beschrieben sind, verabreicht.

Bei einer Ausführungsform der Erfindung wird die Verbindung der Formel I oder II in Kombination mit NPY Inhibitoren (Neuropeptid Y), wie sie z. B. in WO2002047670 beschrieben sind, verabreicht.

Bei einer Ausführungsform der Erfindung wird die Verbindung der Formel I oder II in Kombination mit Natrium/Wasserstoff Austausch Inhibitoren, wie sie z. B. in WO2003092694 beschrieben sind, verabreicht.

Bei einer Ausführungsform der Erfindung wird die Verbindung der Formel I oder II in Kombination mit Modulatoren des Glukocorticoidrezeptors, wie sie z. B. in WO2005090336 beschrieben sind, verabreicht.

Bei einer Ausführungsform der Erfindung wird die Verbindung der Formel I oder II in Kombination mit Nikotin Rezeptor Agonisten, wie sie z. B. in WO2004094429 beschrieben sind, verabreicht.

Bei einer Ausführungsform der Erfindung wird die Verbindung der Formel I oder II in Kombination mit NRIs (Norepinephrine reuptake inhibitors), wie sie z. B. in WO2002053140 beschrieben sind, verabreicht.

Bei einer Ausführungsform der Erfindung wird die Verbindung der Formel I oder II in Kombination mit MOA (E-beta-methoxyacrylate), wie z.B. Segeline oder wie sie z. B. in WO2002053140 beschrieben sind, verabreicht.

Bei einer Ausführungsform der Erfindung wird die Verbindung der Formel I oder II in Kombination mit antithrombotischen Wirkstoffen, wie z. B. Clopidrogel, verabreicht.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

Zu den vorstehend genannten Entwicklungskodes werden nachfolgend teilweise die Formeln aufgeführt.

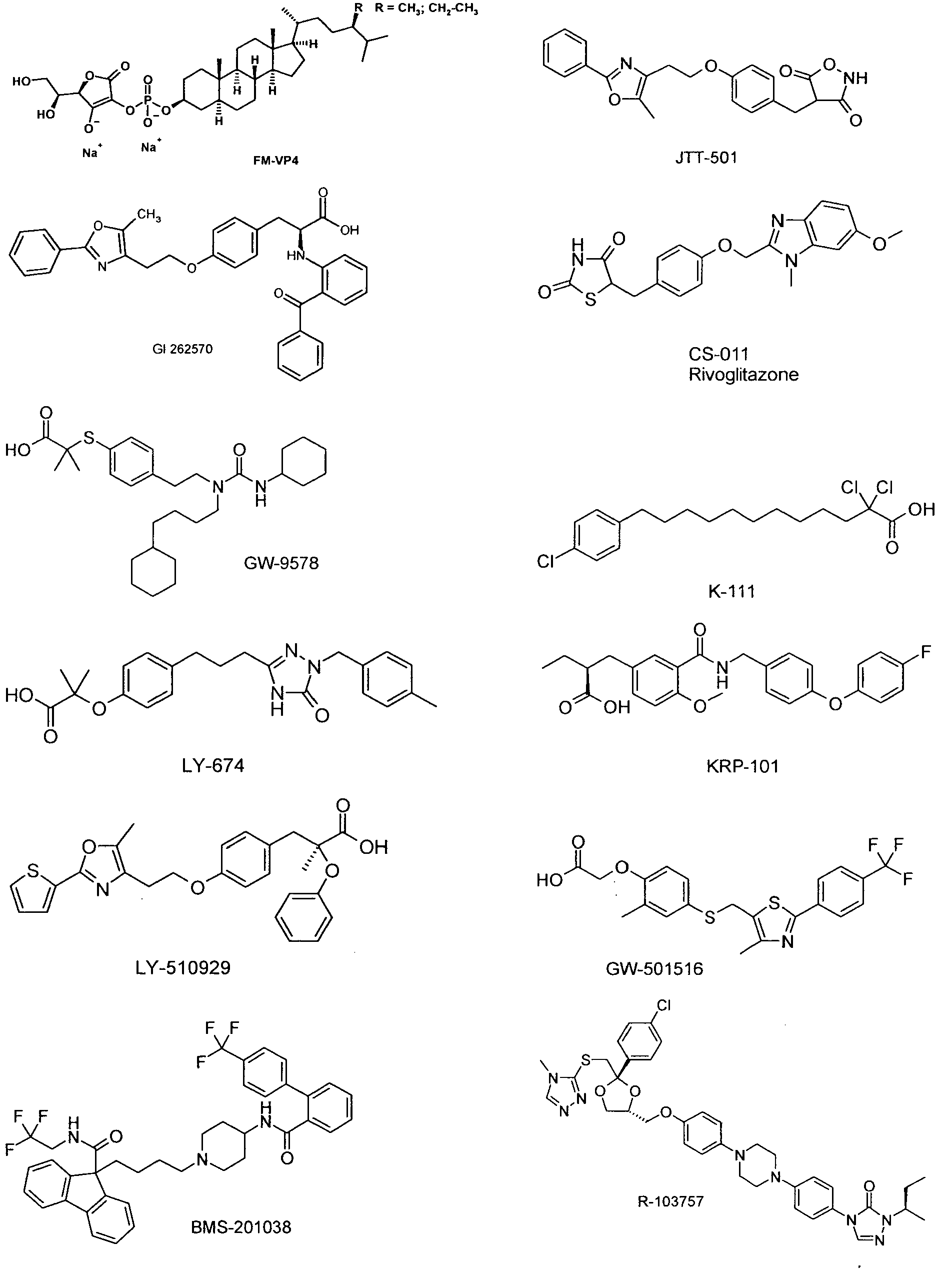

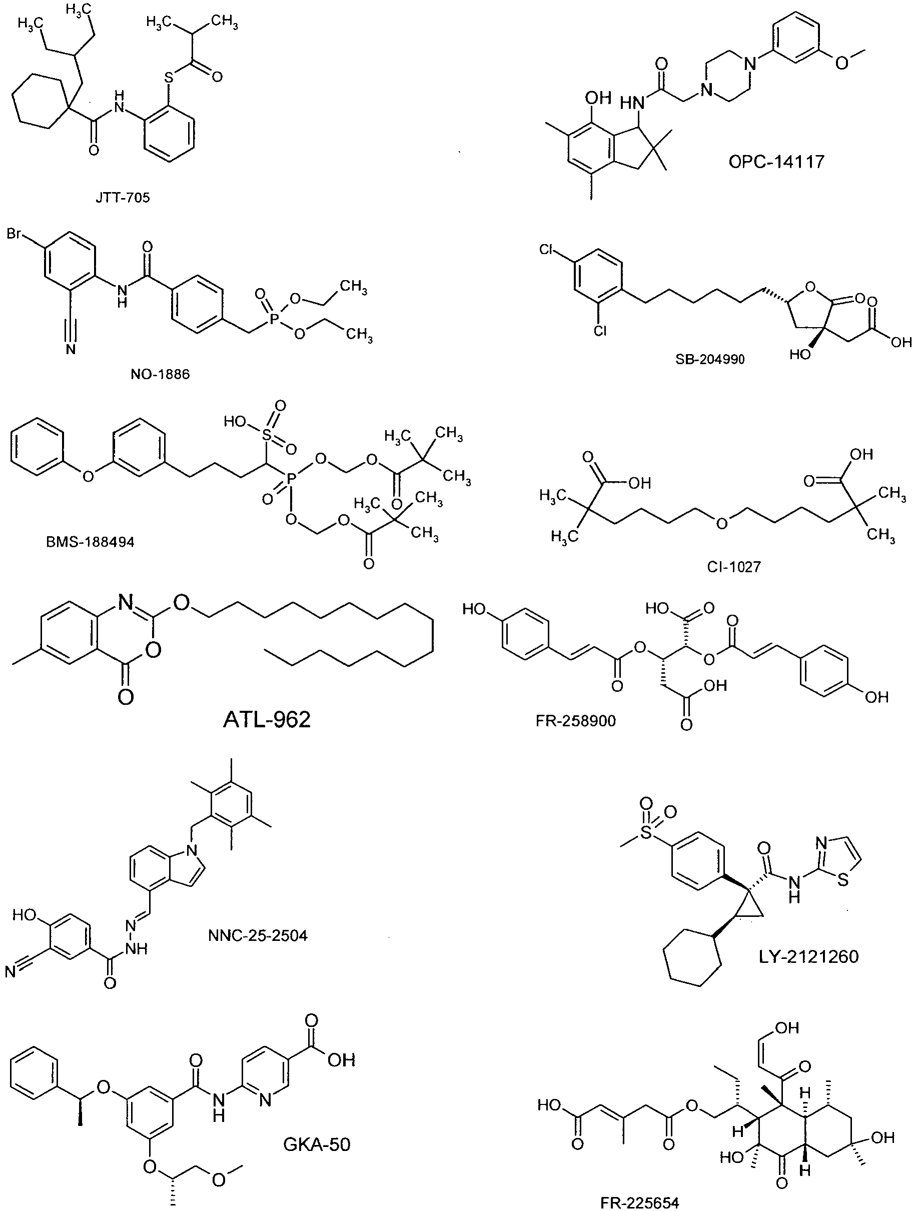

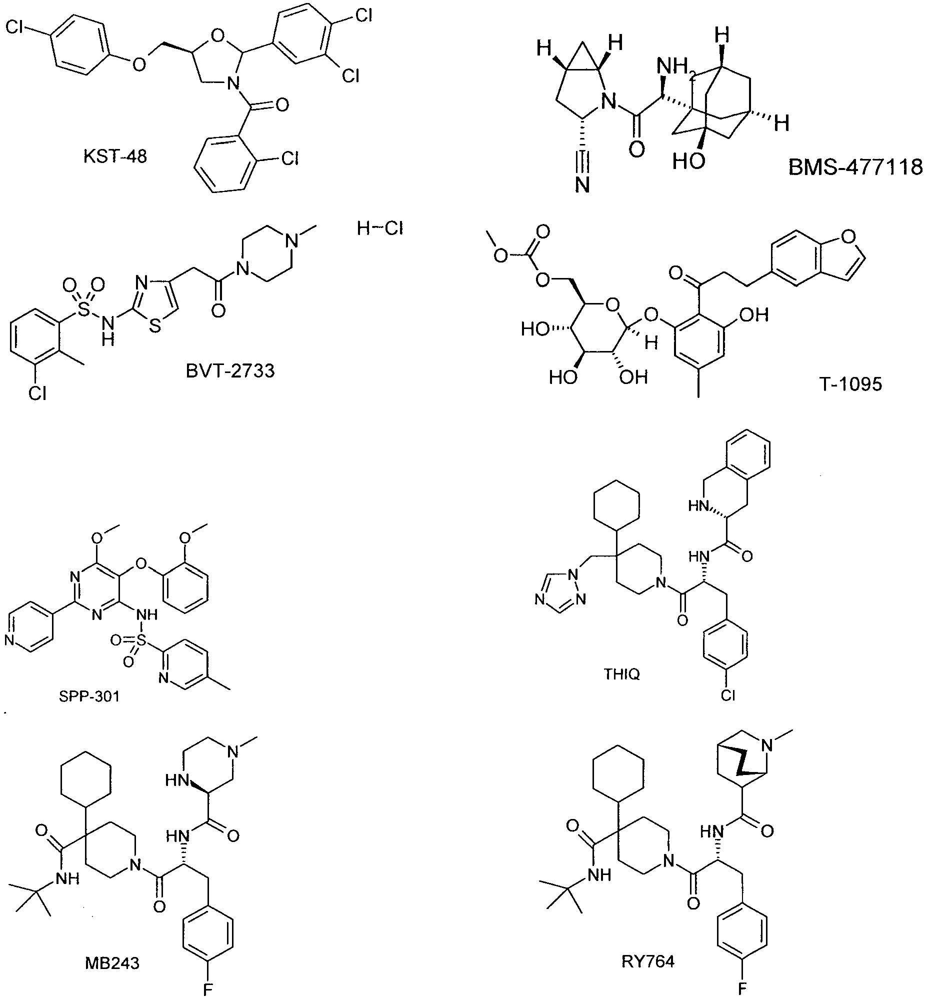

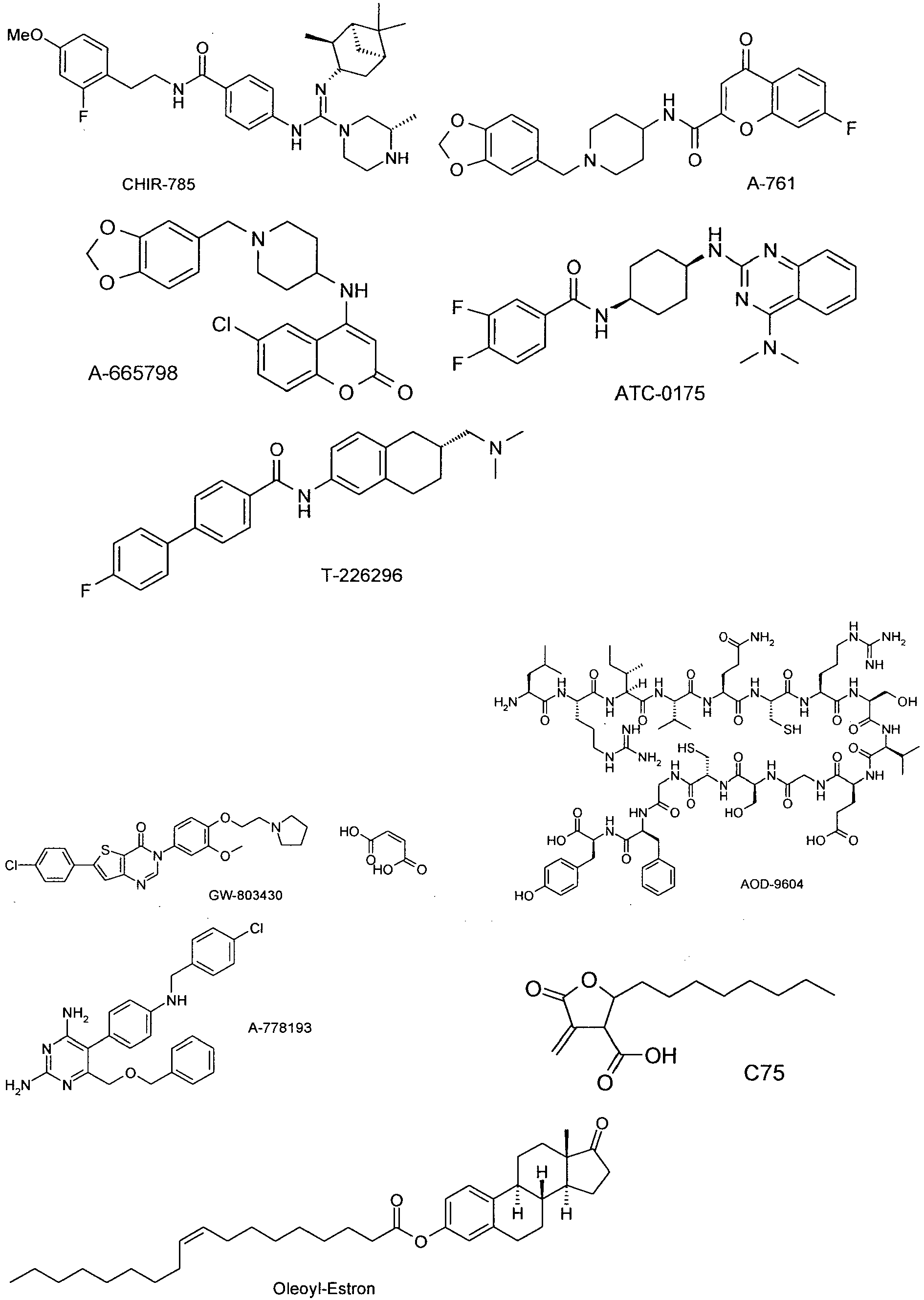

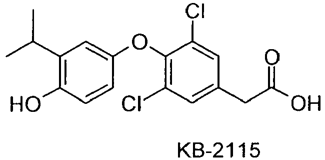

Die Wirksamkeit der erfindungsgemäßen Verbindungen der Formeln I oder II wurde an folgenden Enzymtestsystemen geprüft:

### Test auf Hemmung der HSL

### Präparation der partiell gereinigten HSL:

Isolierte Rattenfettzellen werden aus Nebenhodenfettgewebe von nicht-behandelten männlichen Ratten (Wistar, 220-250 g) durch Kollagenasebehandlung gemäß publizierter Verfahren gewonnen (z.B. S. Nilsson et al., Anal. Biochem. 158, 1986, 399 - 407; G. Fredrikson et al., J. Biol. Chem. 256, 1981, 6311 - 6320; H. Tornquist et al., J. Biol. Chem. 251, 1976, 813 - 819). Die Fettzellen aus 10 Ratten werden dreimal durch Flotation mit jeweils 50 ml Homogenisationspuffer (25 ml Tris/HCl, pH 7.4, 0.25 M Sucrose, 1 mM EDTA, 1 mM DTT, 10 µg/ml Leupeptin, 10 µg/ml Antipain, 20 µg/ml Pepstatin) gewaschen und schließlich in 10 ml Homogenisationspuffer aufgenommen. Die Fettzellen werden im Teflon-in-Glas Homogenisator (Braun-Melsungen) durch 10 Hübe bei 1500 rpm und 15°C homogenisiert. Das Homogenisat wird zentrifugiert (Sorvall SM24-Röhrchen, 5000 rpm, 10 min, 4°C). Der Unterstand zwischen der oben liegenden Fettschicht und dem Pellet wird abgenommen und die Zentrifugation wiederholt. Der daraus resultierende Unterstand wird erneut zentrifugiert (Sorvall SM24-Röhrchen, 20000 rpm, 45 min, 4°C). Der Unterstand wird abgenommen und mit 1 g Heparin-Sepharose (Pharmacia-Biotech, CL-6B, 5 x gewaschen mit 25 mM Tris/HCl, pH 7.4, 150 mM NaCl) versetzt. Nach Inkubation für 60 min bei 4°C (in Intervallen von 15 min aufschütteln) wird der Ansatz zentrifugiert (Sorvall SM24-Röhrchen, 3000 rpm, 10 min, 4°C). Der Überstand wird durch Zugabe von Eisessig auf pH 5.2 gebracht und 30 min bei 4°C inkubiert. Die Präzipitate werden durch Zentrifugation (Sorvall SS34, 12000 rpm, 10 min, 4°C) gesammelt und in 2.5 ml 20 mM Tris/HCl, pH 7.0, 1 mM EDTA, 65 mM NaCl, 13 % sucrose, 1 mM DTT, 10 µg/ml Leupeptin/Pepstatin/Antipain suspendiert. Die Suspension wird über Nacht bei 4°C gegen 25 mM Tris/HCl, pH 7.4, 50 % Glyzerin, 1 mM DTT, 10 µg/ml Leupeptin, Pepstatin, Antipain dialysiert und dann auf eine Hydroxiapatit-Säule aufgetragen (0.1 g pro 1 ml Suspension, äquilibriert mit 10 mM Kaliumphosphat, pH 7.0, 30 % Glyzerin, 1 mM DTT). Die Säule wird mit vier Volumina Äquilibrierungspuffer bei einer Flußrate von 20 bis 30 ml/h gewaschen. Die HSL wird mit einem Volumen Äquilibrierungspuffer, der 0.5 M Kaliumphosphat enthält, eluiert, sodann dialysiert (s.o.) und 5- bis 10-fach konzentriert durch Ultrafiltration (Amicon Diaflo PM 10 Filter) bei 4°C. Die partiell gereinigte HSL kann 4 bis 6 Wochen bei -70°C aufbewahrt werden.

### Assay auf HSL-Aktivität:

Für die Herstellung des Substrats werden 25-50 µCi [3H]Trioleoylglycerin (in Toluol), 6.8 µMol unmarkiertes Trioleoylglycerin und 0.6 mg Phospholipide (Phosphatidylcholin/Phosphatidylinositol 3:1 w/v) gemischt, über N₂ getrocknet und dann in 2 ml 0.1 M KPi (pH 7.0) durch Ultraschallbehandlung (Branson 250, Mikrospitze, Einstellung 1-2, 2 x 1 min im 1-min Intervall) aufgenommen. Nach Zugabe von 1 ml KPi und erneuter Ultraschallbehandlung (4 x 30 sec auf Eis in 30-sec Intervallen) wird 1 ml 20% BSA (in KPi) hinzugefügt (Endkonzentration Trioleoylglyzerin 1.7 mM). Für die Reaktion werden 100 µl Substratlösung zu 100 µl HSL-Lösung (HSL präpariert wie oben, verdünnt in 20 mM KPi, pH 7.0, 1 mM EDTA, 1 mM DTT, 0.02% BSA, 20 µg/ml Pepstatin, 10 µg/ml Leupeptin) pipettiert und für 30 min bei 37°C inkubiert. Nach Zugabe von 3.25 ml Methanol/Chloroform/Heptan (10:9:7) und von 1.05 ml 0.1 M K2CO3, 0.1 M Borsäure (pH 10.5) wird gut gemischt und schließlich zentrifugiert (800 x g, 20 min). Nach Phasentrennung wird ein Äquivalent der oberen Phase (1 ml) abgenommen und die Radioaktivität durch Flüssigszintillationsmessung bestimmt.
Substanzen werden üblicherweise in vier unabhängigen Ansätzen geprüft. Die Hemmung der enzymatischen Aktivität der HSL durch eine Testsubstanz wird durch den Vergleich mit einer nicht-gehemmten Kontrollreaktion bestimmt. Die Berechnung des IC₅₀-Wertes erfolgt über eine Hemmkurve mit mind. 10 Konzentrationen der Testsubstanz. Für die Analyse der Daten wird das Softwarepaket GRAPHIT, Elsevier-BIOSOFT, benutzt.

### Test auf Hemmung der EL:

EL wird als sekretorisches Protein von rekombinanten Zell-Linien (CHO, HEK293) in hoher Konzentration in Zellkulturmedium abgegeben (konditioniertes Medium). Dieses wird nach Aufkonzentration als Enzymlösung eingesetzt.

Zur Charakterisierung der enzymatischen Aktivität von endothelialer Lipase und der Wirkung von Inhibitoren wird das Phospholipase-spezifische Substrat 1,2-bis-(4,4-difluoro-5,7-dimethyl-4-bora-3a,4a-diaza-s-indacene-3-undecanoyl)-sn-glycero-3-phosphocholine, (Hersteller Molecular Probes) verwendet. Durch Hydrolyse der A1 Esterbindung dieses Phospholipids durch das Enzym wird der Fluoreszenzfarbstoff Bodipy freigesetzt, der nach Trennung durch Dünnschichtchromatographie auf einer HPTLC-Platte (Kieselgel 60, Merck) oder direkt im Reaktionsgefäß durch Messen der Fluoreszenz nachgewiesen werden kann.
Zur Herstellung der Substratlösung werden 100 µg 1,2-bis-(4,4-difluoro-5,7-dimethyl-4-bora-3a,4a-diaza-s-indacene-3-undecanoyl)-*sn*-glycero-3-phospho-choline (Hersteller Molecular Probes), 2,4 mg Tripalmitin (Sigma) und 7,9 mg DOP - Cholin (1,2-Dioleoyl-sn-glycero-3-phosphocholin) in 393 µl Chloroform aufgenommen und im Anschluss 157 µl in ein frisches Reaktionsgefäß überführt. Nach Abdampfen des Lösungsmittels wird das Lipidgemisch in 4 ml 200 mM TRIS-HCl, 150 mM Natriumchlorid, pH = 7,4 durch zweimaliges Sonifizieren gelöst. Die anschließende Enzymreaktion erfolgt für 60 Minuten bei 37°C. Hierzu werden 45 µl der Substratlösung mit 1 µl Inhibitor entsprechender Konzentration (gelöst in DMSO, zur Kontrolle wird reine DMSO-Lösung verwendet) und 5 µl Enzymlösung (konditioniertes Medium) inkubiert. Im Anschluß werden 3 µl des Testansatzes auf eine HPTLC-Platte (Kieselgel 60, Merck) aufgetragen und der freigesetzte Fluoreszenzfarbstoff zum Nachweis mit einem Fließmittel (Diäthylether : Petroleumbenzin : Essigsäure [78:22:1]) getrennt. Nach dem Abdampfen des Fließmittels wird die Platte in einen Fluoreszenz-Scanner eingelesen. Als Maß für die Enzymaktivität ist eine gesteigerte Freisetzung des Fluoreszenzfarbstoffes in der ungehemmten Reaktion zu beobachten.
In Abhängigkeit der verwendeten Inhibitorkonzentration ergibt sich eine Reduktion der enzymatischen Aktivität, die Inhibitiorkonzentration, bei welcher eine halbmaximale Enzymaktivität beobachtet wird, wird als IC₅₀ bezeichnet.

In diesen Tests zeigten die Verbindungen der Beispiele folgende von IC₅₀ -Werte:

| Beispiel | IC₅₀ [µM] HSL | IC₅₀ [µM] EL |
|---|---|---|
| 1a | 0.32 | |
| 1b | 0.62 | |
| 8b | 1.15 | |
| 9a | | 0.05 |
| 9b | | 0.06 |
| 10a | | 0.26 |
| 10b | | 1.47 |
| 11 | | 0.22 |
| 12 | | 0.84 |
| 13 | 1.0 | |
| 14 | 1.5 | |
| 15 | 0.58 | |

### Verfahren zur Herstellung

Die Herstellung der erfindungsgemäßen Verbindungen der allgemeinen Formeln I oder II erfolgt nach an und für sich bekannten Methoden z.B. durch Acylierung von substituierten, bzw. unsubstituiertem Indazol-derivaten III mit Carbamoylchloriden IV (Methode A), oder in zwei Stufen durch Umsetzung von Indazol-derivaten III mit Phosgen oder Äquivalenten wie Chlorcarbonsäuretrichlormethylester, Carbonsäureditrichlormethylester oder Chlorameisensäure-4-nitrophenylester und weiterer Umsetzung des erhaltenen Indazol-carbonsäurederivats mit einem Amin V (Methode B). Für Verbindungen bei denen R2 Wasserstoff ist, können die Indazolderivaten III auch mit den entsprechenden Isocyanaten VI R1-N=C=O umgesetzt werden.

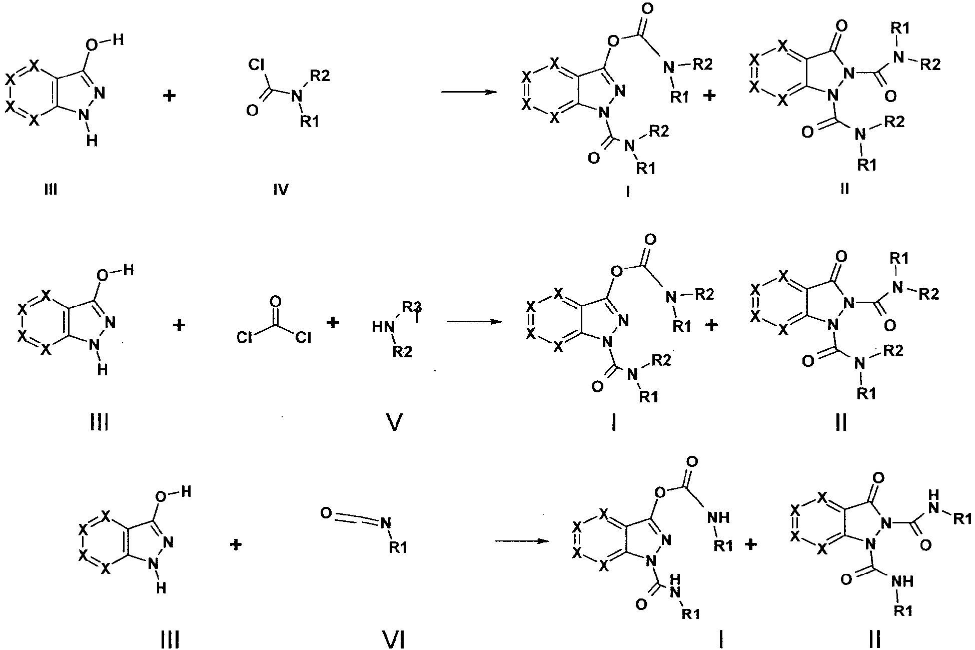

Da bei diesen Reaktionen in der Regel Säuren freigesetzt werden, empfiehlt es sich zur Beschleunigung Basen wie Pyridin, Triethylamin, Natronlauge oder Alkalicarbonate zuzusetzen. Die Reaktionen können in weiten Temperaturbereichen durchgeführt werden. In der Regel hat es sich als vorteilhaft herausgestellt, bei 0°C bis zum Siedepunkt des verwendeten Lösungsmittels zu arbeiten. Als Lösemittel kommen beispielsweise Methylenchlorid, THF, DMF, Toluol, Essigester, n-Heptan, Dioxan, Diethylether oder Pyridin zum Einsatz. Wenn unter wasserfreien Bedingungen gearbeitet wird, haben sich auch starke Basen wie Lithiumhydrid, Natriumhydrid oder Kalium-tert.-butylat in aprotischen Lösungsmitteln wie THF oder DMF bewährt.
Die als Ausgangsverbindungen III eingesetzten Indazol-derivate oder entsprechenden Aza-substituierten Derivate sind kommerziell erhältlich oder lassen sich nach Literatur bekannten Verfahren herstellen (z.B. L. Baiocchi, G. Corsi Synthesis (1978), 633-648, I. Sekikawa et al. J. Het. Chem. (1973), 931-932).

Die nach oben beschriebenen Verfahren erhaltenen Verbindungen der Formel I und II können durch bekannte Trennverfahren, wie beispielsweise chromatographische Verfahren getrennt werden.
Die nachfolgend aufgeführten Beispiele dienen zur Erläuterung der Erfindung, ohne diese jedoch einzuschränken.

### Beispiele

### Beispiel 1:

### 4-Phenyl-piperazin-1-carbonsäure 6-fluoro-1-(4-phenyl-piperazin-1-carbonyl)-1 H-indazol-3-yl ester (1a) und 6-Fluoro-1,2-bis-(4-phenyl-piperazin-1-carbonyl)-1,2-dihydro-indazol-3-on (1b)

200 mg (1.315 mmol) 6-Fluoro-1H-indazol-3-ol wurden in 5 ml Pyridin gelöst. Nach Zugabe von 365 µl (2.63 mmol) Trietylamin und 355 mg (1.56 mmol) 4-Phenyl-piperazin-1-carbonylchlorid wurde 3 h bei Raumtemperatur gerührt. Nach Zugabe von 730 µl Triethylamin und 177 mg 4-Phenyl-piperazin-1-carbonylchlorid wurde 20 h weitergerührt. Das Reaktionsgemisch wurde eingeengt, mit Wasser und Ethylacetat versetzt und die organische Phase abgetrennt, eingeengt und über präparative HPLC (PR18, Acetonitril/Wasser 0.1% TFA) gereinigt. Ausbeute: 12 mg (2 %), M+H+: 529.34, 4-Phenyl-piperazin-1-carbonsäure 6-fluoro-1-(4-phenyl-piperazin-1-carbonyl)-1 H- indazol-3-yl ester; 30 mg, (4%), M+H+: 529.40, 6-Fluoro-1,2-bis-(4-phenyl-piperazin-1-carbonyl)-1,2-dihydro-indazol-3-on.
Ausserdem konnte noch 4-Phenyl-piperazin-1-carbonsäure 6-fluoro-1 H-indazol-3-yl ester als nicht erfindungsgemäße Verbindung isoliert werden.

### Beispiel 2:

### Thiomorpholin-4-carbonsäure 6-fluoro-1-(thiomorpholin-4-carbonyl)-1 H-indazol-3-yl ester

Analog Beispiel 1 wurden 200 mg (1.315 mmol) 6-Fluoro-1H-indazol-3-ol mit 261.4 mg (1.578 mmol) Thiomorpholin-4-carbonylchlorid umgesetzt. Ausbeute: 39 mg (7 %), M+H+: 411.17.

### Beispiel 3:

### 4-Trifluoromethyl-piperidin-1-carbonsäure 6-fluoro-1-(4-trifluoromethyl-piperidin-1-carbonyl)-1 H-indazol-3-yl ester

Analog Beispiel 1 wurden 200 mg (1.315 mmol) 6-Fluoro-1H-indazol-3-ol mit 340 mg (1.578 mmol) 4-Trifluoromethyl-piperidin-1-carbonylchlorid umgesetzt. Ausbeute: 8 mg (1 %), M+H+: 511.36.

### Beispiel4:

### 2,6-Dimethyl-morpholin-4-carbonsäure 1-(2,6-dimethyl-morpholin-4-carbonyl)-6-fluoro-1 H-indazol-3-yl ester

Analog Beispiel 1 wurden 200 mg (1.315 mmol) 6-Fluoro-1H-indazol-3-ol mit 280.3 mg (1.578 mmol) 2,6-Dimethyl-morpholin-4-carbonylchlorid umgesetzt. Ausbeute: 16 mg (3 %), M+H+: 435.29.

### Beispiel 5:

### 4-Methyl-piperidin-1-carbonsäure 4,6-difluoro-1-(4-methyl-piperidin-1-carbonyl)- 1H-indazol-3-yl ester und 4,6-Difluoro-1,2-bis-(4-methyl-piperidin-1-carbonyl)-1,2-dihydro-indazol-3-on

Analog Beispiel 1 wurden 60 mg (0.35 mmol) 4,6-Difluoro-1H-indazol-3-ol mit 85.6 mg (0.53 mmol) 4-Methyl-piperidin-1-carbonylchlorid umgesetzt. Ausbeute: 73 mg (49 %), M+H+: 421.35 4-Methyl-piperidin-1-carbonsäure 4,6-difluoro-1-(4-methyl-piperidin-1-carbonyl)- 1 H-indazol-3-yl ester und 3 mg (2%), M+H+: 421.34 4,6-Difluoro-1,2-bis-(4-methyl-piperidin-1-carbonyl)-1,2-dihydro-indazol-3-on.

### Beispiel 6:

### 4-Methyl-piperidin-1-carbonsäure 1-(4-methyl-piperidin-1-carbonyl)-6- trifluoromethyl-1 H-pyrazolo[3,4-b]pyridin-3-yl ester

Analog Beispiel 1 wurden 120 mg (0.59 mmol) 6-Trifluoromethyl-1H-pyrazolo[3,4-b]pyridin-3-ol mit 143.3 mg (0.89 mmol) 4-Methyl-piperidin-1-carbonylchlorid umgesetzt. Ausbeute: 71 mg (26 %), M+H+: 454.27.

### Beispiel 7:

### 4-Methyl-piperidin-1-carbonsäure 6-chloro-4-methyl-1-(4-methyl-piperidin-1- carbonyl)-1 H-pyrazolo[3,4-b]pyridin-3-yl ester

Analog Beispiel 1 wurden 1.2 g (6.54 mmol) 6-Chloro-4-methyl-1 H-pyrazolo[3,4-b]pyridin-3-ol mit 1.29 g (7.98 mmol) 4-Methyl-piperidin-1-carbonylchlorid umgesetzt. Ausbeute: 238 mg (8 %), M+H+: 434.27.

### Beispiel 8:

### 6-Fluoro-1,2-bis-(4-methyl-piperidin-1-carbonyl)-1,2-dihydro-indazol-3-on (8a) und 4-Methyl-piperidin-1-carbonsäure 6-fluoro-1-(4-methyl-piperidin-1-carbonyl)-1 H- indazol-3-yl ester (8b)

Analog Beispiel 1 wurden 10 g (65.73 mmol) 6-Fluoro-1H-indazol-3-ol mit 11.69 g (72.3 mmol) 4-Methyl-piperidin-1-carbonylchlorid umgesetzt. Ausbeute: 455 mg (1.7 %), M+H+: 403.14, 6-Fluoro-1,2-bis-(4-methyl-piperidin-1-carbonyl)-1,2-dihydro-indazol-3-on und 1.6 g (6 %), M+H+: 403.18, 4-Methyl-piperidin-1-carbonsäure 6-fluoro-1-(4-methyl-piperidin-1-carbonyl)-1 H- indazol-3-yl ester.

### Beispiel 9

### 6-Chloro-3-oxo-3H-indazol-1,2-dicarbonsäure bis-hexylamid (10a) und Hexylcarbaminsäure 6-chloro-1-hexylcarbamoyl-1 H-indazol-3-yl ester (10b)

100 mg (0.59 mmol) 6-Chloro-1H-indazol-3-ol wurden in 5 ml DMF gelöst. Nach Zugabe von 83 mg (0.65 mmol) 1-Isocyanatohexan wurde 1.5 h bei Raumtemperatur und 1 h bei 50°C gerührt, eingeengt und über präparative HPLC (PR18, Acetonitril/Wasser 0.1% TFA) gereinigt. Ausbeute: 17 mg (7 %), M+H+: 296.14, 6-Chloro-3-oxo-3H-indazol-1,2-dicarbonsäure bis-hexylamid und 12 mg (5 %) Hexylcarbaminsäure 6-chloro-1-hexylcarbamoyl-1H-indazol-3-yl ester. Ausserdem konnte noch 6-Chloro-3-hydroxy-indazol-1-carbonsäure hexylamid als nicht erfindungsgemäße Verbindung isoliert werden.

### Beispiel 10

### 6-Chloro-3-oxo-3H-indazol-1,2-dicarbonsäure bis-benzylamid (10a) und Benzylcarbaminsäure 1-benzylcarbamoyl-6-chloro-1 H-indazol-3-yl ester (10b)

Analog Beispiel 9 wurden 100 mg (0.59 mmol) 6-Chloro-1H-indazol-3-ol mit 86.85 mg (0.65 mmol) Isocyanatomethylbenzen umgesetzt. Ausbeute: 31 mg (12 %) 6-Chloro-3-oxo-3H-indazol-1,2-dicarbonsäure bis-benzylamid, und 15 mg (6 %) Benzylcarbaminsäure 1-benzylcarbamoyl-6-chloro-1 H-indazol-3-yl ester.

### Beispiel 11:

### 1-(4-Methyl-piperidin-1-carbonyl)-3-oxo-1,3-dihydro-indazol-2-carbonsäure 2-methylbenzylamid

105 mg (0.78 mmol) 1H-Indazol-3-ol wurden in 3 ml THF mit 138.3 mg (0.94 mmol) 1-Isocyanatomethyl-2-methyl-benzen zu 3-Oxo-1,3-dihydro-indazol-2-carbonsäure 2-methyl-benzylamid umgesetzt. 50 mg (178 µmol) dieser Verbindung wurden in 3 ml Pyridin bei Raumtemperatur mit 40 mg (355 µmol) Triethylamin und 43 mg (266 µmmol) 4-Methyl-piperidin-1-carbonylchlorid 7 h gerührt. Nach Zugabe von weiteren 120 mg 4-Methyl-piperidin-1-carbonylchlorid und 100 mg Triethylamin in 3 Portionen wurde nochmals 7 h gerührt, eingeengt und über präparative HPLC (PR18, Acetonitril/Wasser 0.1 % TFA) gereinigt. Ausbeute: 48 mg (66 %), M+H+: 407.15.

### Beispiel 12

### 3-Oxo-3H-indazol-1,2-dicarbonsäure bis-(2-methyl-benzylamid)

Analog Beispiel 10 wurden 300 mg (2.24 mmol) 1H-Indazol-3-ol mit 395.5 mg (2.69 mmol) 1-Isocyanatomethyl-2-methyl-benzen umgesetzt. Ausbeute: 177 mg (18 %), M+H+: 429.26.

### Beispiel 13:

### 4-Methyl-piperidin-1-carbonsäure 6-amino-1-(4-Trifluoromethyl-piperidin-1-carbonyl)-1 H-indazol-3-yl ester

Analog Beispiel 11 wurden 35.66 mg (0.13 mmol) 4-Methyl-piperidin-1-carbonsäure 6-amino-1H-indazol-3-yl ester mit 33.63 mg (0.16 mmol) 4-Trifluoromethyl-piperidin-1-carbonylchlorid umgesetzt. Ausbeute: 10 mg (17 %), M+H+: 454.13.

### Beispiel 14

### 4-Trifluoromethyl-piperidin-1-carbonsäure 6-hydroxy-4-methyl-1-(4-methyl-piperazin-1-carbonyl)-1H-pyrazolo[3,4-b]pyridin-3-yl ester; als Trifluoroacetat

Analog Beispiel 11 wurden 79.88 mg (0.23 mmol) 4-Trifluoromethyl-piperidin-1-carbonsäure 6-hydroxy-4-methyl-1H-pyrazolo[3,4- b]pyridin-3-yl ester mit 41.3 mg (0.2 mmol) 4-Methyl-piperazin-1-carbonylchlorid-hydrochlorid umgesetzt. Ausbeute: 18 mg (16 %), M+H+: 471.35.

### Beispiel 15

### 4-Methyl-piperidin-1-carbonsäure 6-hydroxy-4-methyl-1-(4-trifluoromethyl-piperidin- 1-carbonyl)-1 H-pyrazolo[3,4-b]pyridin-3-yl ester

Analog Beispiel 11 wurden 99.87 mg (0.344 mmol) 4-Methyl-piperidin-1-carbonsäure 6-hydroxy-4-methyl-1 H-pyrazolo[3,4-b]pyridin-3- yl-ester mit 83.4 mg (0.52 mmol) 4-Trifluoromethyl-piperidin-1-carbonylchlorid umgesetzt. Ausbeute: 16 mg (10 %), M+H+: 470.20.

## Patentansprüche

1. Verbindung der allgemeinen Formeln I oder II 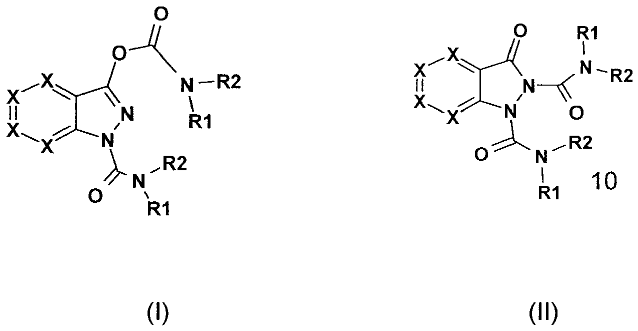 worin bedeuten
X gleich oder verschieden =C(-R)- oder =N-, wobei höchstens ein X für =N- steht;
R gleich oder verschieden Wasserstoff, Halogen, Hydroxy, (C₁-C₆)-Alkyl, Trifluormethyl, (C₁-C₃)-Alkyloxy oder Amino;
R1 gleich oder verschieden (C₆-C₁₀)-Alkyl oder Y-Phenyl, das durch Halogen, (C₁-C₆)-Alkyl, (C₁-C₃)-Alkyloxy oder Trifluormethyl substituiert sein kann;
Y CH₂;
R2 Wasserstoff; oder
R1 und R2 zusammen mit dem sie tragenden Stickstoffatom ein monocyclisches, gesättigtes 5- bis 6-gliedriges Ringsystem bilden, deren einzelne Glieder durch ein bis drei Atome oder Atomgruppen aus der Reihe -CHR4-, - NR4-, -O-, -S- ersetzt sein können, mit der Maßgabe, dass zwei Einheiten aus der Reihe -O-, -S- nicht benachbart sein dürfen;
R4 (C₁-C₆)-Alkyl, Trifluormethyl oder Phenyl bedeuten,
die tautomeren Formen der Verbindungen sowie deren physiologisch verträgliche Salze.

2. Verbindung der Formeln I oder II gemäß Anspruch 1, **dadurch gekennzeichnet, dass**
NR1R2 für ein monocyclisches gesättigtes 5- bis 6-gliedriges Ringsystem steht, das in 4 Position ein Atom oder Atomglied aus der Reihe -CHR4-,-CR4R5-, -(C=R4)-, -NR4-, -O-, -S- enthält.

3. Verbindung der Formeln I oder II gemäß Anspruch 1, **dadurch gekennzeichnet, dass**
X gleich oder verschieden =C(-R)- oder =N-, wobei höchstens ein X für =N- steht;
R gleich oder verschieden Wasserstoff, F, Cl, Hydroxy, Methyl, Trifluormethyl oder Amino;
R1 gleich oder verschieden (C₆-C₁₀)-Alkyl oder Y-Phenyl, welches durch Methyl substituiert sein kann;
Y CH₂;
R2 Wasserstoff; oder
R1 und R2 zusammen mit dem sie tragenden Stickstoffatom ein monocyclisches, gesättigtes 5- bis 6-gliedriges Ringsystem bilden, deren einzelne Glieder durch ein bis drei Atome oder Atomgruppen aus der Reihe -CHR4-, - NR4-, -O-, -S- ersetzt sein können, mit der Maßgabe, dass zwei Einheiten aus der Reihe -O-, -S- nicht benachbart sein dürfen;
R4 Methyl, Trifluormethyl oder Phenyl, welches durch Methyl oder Cl ein oder zweifach substituiert sein kann;
bedeuten;
die tautomeren Formen der Verbindungen sowie deren physiologisch verträgliche Salze.

4. Verbindung der Formeln I oder II gemäß Ansprüchen 1, 2 oder 3, **dadurch gekennzeichnet, dass**
NR1R2 für Piperidin steht, das in 4 Position das Atomglied -CHR4- enthält.

5. Verbindung der Formeln I oder 11 gemäß Ansprüchen 1 bis 4, **dadurch gekennzeichnet, dass**
X gleich oder verschieden =C(-R)- bedeutet.

6. Verbindung der Formeln I oder II gemäß Ansprüchen 1 bis 4, **dadurch gekennzeichnet, dass**
X in Position 4, 5 und 6 für gleich oder verschieden =C(-R)-, in Position 7 für =N- steht.

7. Verbindung der Formeln I oder II gemäß Ansprüchen 1 bis 5, **dadurch gekennzeichnet, dass**
X in Position 4, 5 und 7 für =C(-R)- mit R = Wasserstoff steht, in Position 6 R nicht für Wasserstoff steht.

8. Arzneimittel enthaltend eine oder mehrere Verbindungen der Formeln I oder II gemäß den Ansprüchen 1 bis 7.

9. Verbindung der Formel I oder II gemäß den Ansprüchen 1 bis 7 zur Behandlung und/oder Prävention von Störungen des Fettsäurestoffwechsels und Glucoseverwertungsstörungen.

10. Verbindung der Formel I oder II gemäß den Ansprüchen 1 bis 7 zur Behandlung und/oder Prävention von Störungen, bei denen Insulin Resistenz eine Rolle spielt.

11. Verbindung der Formel I oder II gemäß den Ansprüchen 1 bis 7 zur Behandlung und/oder Prävention von Diabetes mellitus und der damit verbundenen Folgeerkrankungen.

12. Verbindung der Formel I oder II gemäß den Ansprüchen 1 bis 7 zur Behandlung und/oder Prävention von Dyslipidämien und deren Folgen.

13. Verbindung der Formel I oder II gemäß den Ansprüchen 1 bis 7 zur Behandlung und/oder Prävention von Zuständen, die mit dem Metabolischen Syndrom assoziert sind.

14. Verbindung der Formel I oder II gemäß den Ansprüchen 1 bis 7 zur Behandlung und/oder Prävention von Zuständen, die mit erniedrigtem HDL-Spiegel verbunden sind.

15. Verbindung der Formel I oder II gemäß den Ansprüchen 1 bis 7 zur Behandlung und/oder Prävention von atherosklerotischen Erkrankungen.

16. Verbindung der Formel I oder II gemäß den Ansprüchen 1 bis 7 in Kombination mit mindestens einem weiteren Wirkstoff zur Behandlung und/oder Prävention von Störungen, bei denen Insulin Resistenz eine Rolle spielt.

17. Verfahren zur Herstellung eines Arzneimittels enthaltend eine oder mehrere der Verbindungen der Formeln I oder II gemäß den Ansprüchen 1 bis 7, **dadurch gekennzeichnet, dass** diese mit einem pharmazeutisch geeigneten Träger vermischt wird und diese Mischung in eine für die Verabreichung geeignete Form gebracht wird.

18. Verfahren zur Herstellung von Verbindungen der allgemeinen Formeln I und/oder II gemäß den Ansprüchen 1 bis 7, **dadurch gekennzeichnet, dass** Indazol-derivate der Formel III
a) mit Carbamoylchloriden der Formel IV acyliert werden;
oder
b) in zwei Stufen zunächst mit Phosgen oder Äquivalenten wie Chlorcarbonsäuretrichlormethylester, Carbonsäureditrichlormethylester oder Chlorameisensäure-4-nitrophenylester und einem zweiten Schritt mit einem Amin V umgesetzt werden,
worin die Substituenten die oben genannten Bedeutungen haben,
und anschließend die Trennung der Verbindungen I und II durch bekannte Verfahren vorgenommen wird.

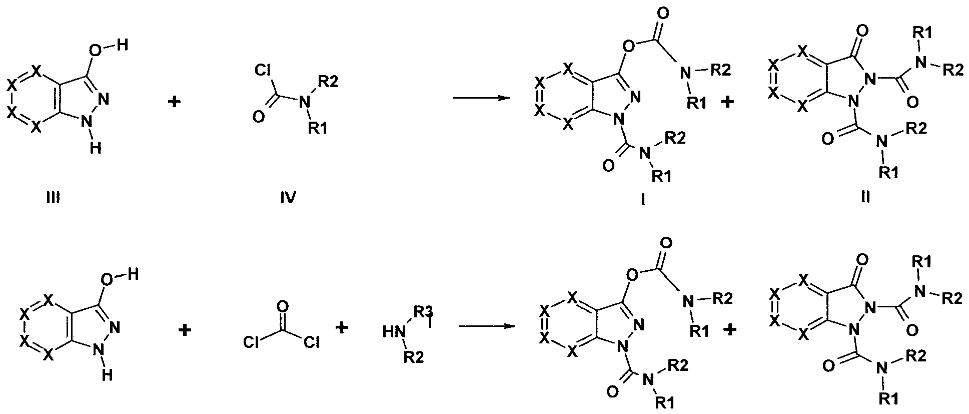

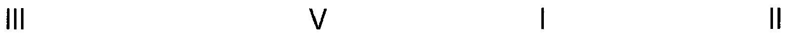

19. Verfahren zur Herstellung von Verbindungen der allgemeinen Formeln I und/oder II mit R2 Wasserstoff gemäß den Ansprüchen 1 bis 4, **dadurch gekennzeichnet, dass** Indazol-derivate der Formel III mit Isocyanaten der Formel VI: O=C=N-R1 umgesetzt werden, und anschließend die Trennung der Verbindungen I und II durch bekannte Verfahren vorgenommen wird.

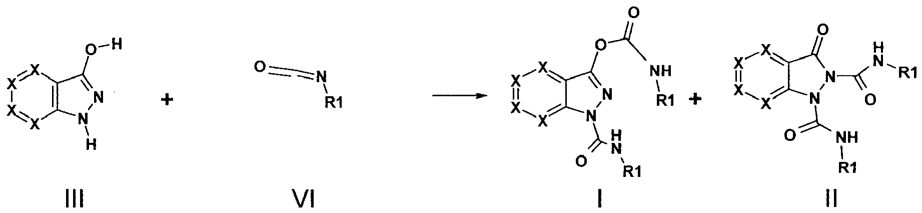

## Claims

1. A compound of the formulae I or II 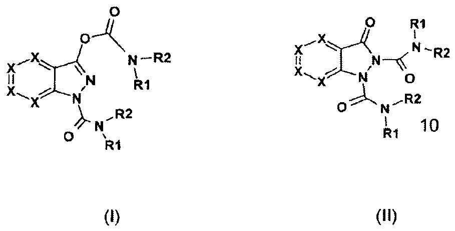 where the meanings are
X identically or differently =C(-R)- or =N-, where not more than one X is =N-;
R identically or differently hydrogen, halogen, hydroxy, (C₁-C₆)-alkyl, trifluoromethyl, (C₁-C₃)-alkyloxy or amino;
R1 identically or differently (C₆-C₁₀)-alkyl or Y-phenyl, which may be substituted by halogen, (C₁-C₆)-alkyl, (C₁-C₃) -alkyloxy, or trifluoromethyl;
Y CH₂;
R2 hydrogen; or
R1 and R2 together with the nitrogen atom carrying them form a monocyclic, saturated 5- to 6-membered ring system, whose individual members may be replaced by one to three atoms or atomic groups from the series -CHR4-, -NR4-, -O-,-S-, with the proviso that two units from the series -O-, -S- may not be adjacent;
R4 (C₁-C₆)-alkyl, trifluoromethyl or phenyl,
the tautomeric forms of the compounds and the physiologically tolerated salts thereof.

2. A compound of the formulae I or II as claimed in claim 1, wherein
NR1R2 is a monocyclic, saturated 5- to 6-membered ring system which contains an atom or atomic member from the series -CHR4-, -CR4R5-, -(C=R4)-, -NR4-, -O-, -S- in position 4.

3. A compound of the formulae I or II as claimed in claim 1, wherein
X is identically or differently =C(-R)- or =N-, where not more than one X is =N-;
R is identically or differently hydrogen, F, Cl, hydroxy, methyl, trifluoromethyl or amino;
R1 is identically or differently (C₆-C₁₀)-alkyl or Y-phenyl, which may be substituted by methyl;
Y is CH₂;
R2 is hydrogen; or
R1 and R2 together with the nitrogen atom carrying them form a monocyclic, saturated 5- to 6-membered ring system, whose individual members may be replaced by one to three atoms or atomic groups from the series -CHR4-, - NR4-, -O-, -S-, with the proviso that two units from the series -O-, -S- may not be adjacent;
R4 is methyl, trifluoromethyl or phenyl, which may be substituted once or twice by methyl or Cl;
the tautomeric forms of the compounds and the physiologically tolerated salts thereof.

4. A compound of the formulae I or II as claimed in claims 1, 2 or 3, wherein
NR1R2 is piperidine which contains the atomic member -CHR4- in position 4.

5. A compound of the formulae I or II as claimed in claims 1 to 4, wherein
X is identically or differently =C(-R)-.

6. A compound of the formulae I or II as claimed in claims 1 to 4, wherein
X in position 4, 5 and 6 is identically or differently =C(-R)-, and in position 7 is =N-.

7. A compound of the formulae I or II as claimed in claims 1 to 5, wherein
X in position 4, 5 and 7 is =C(-R)- with R = hydrogen, and in position 6 R is not hydrogen.

8. A medicament comprising one or more compounds of the formulae I or II as claimed in claims 1 to 7.

9. A compound of the formula I or II as claimed in claims 1 to 7 for the treatment and/or prevention of disorders of fatty acid metabolism and glucose utilization disorders.

10. A compound of the formula I or II as claimed in claims 1 to 7 for the treatment and/or prevention of disorders in which insulin resistance is involved.

11. A compound of the formula I or II as claimed in claims 1 to 7 for the treatment and/or prevention of diabetes mellitus and the sequelae associated therewith.

12. A compound of the formula I or II as claimed in claims 1 to 7 for the treatment and/or prevention of dyslipidemias and their sequelae.

13. A compound of the formula I or II as claimed in claims 1 to 7 for the treatment and/or prevention of conditions associated with the metabolic syndrome.

14. A compound of the formula I or II as claimed in claims 1 to 7 for the treatment and/or prevention of conditions associated with reduced HDL level.

15. A compound of the formula I or II as claimed in claims 1 to 7 for the treatment and/or prevention of atherosclerotic disorders.

16. A compound of the formula I or II as claimed in claims 1 to 7 in combination with at least one further active ingredient for the treatment and/or prevention of disorders in which insulin resistance is involved.

17. A process for producing a medicament comprising one or more of the compounds of the formulae I or II as claimed in claims 1 to 7, which comprises mixing the latter with a pharmaceutically suitable carrier and converting this mixture into a form suitable for administration.

18. A process for preparing compounds of the formulae I and/or II as claimed in claims 1 to 7, which comprises indazole derivatives of the formula III
a) being acylated with carbamoyl chlorides of the formula IV;
or
b) in two stages being reacted initially with phosgene or equivalents such as trichloromethyl chlorocarbonate, ditrichloromethyl carbonate or 4-nitrophenyl chloroformate and in a second step with an amine V,
in which the substituents have the abovementioned meanings,
and subsequently separating the compounds I and II by known processes.

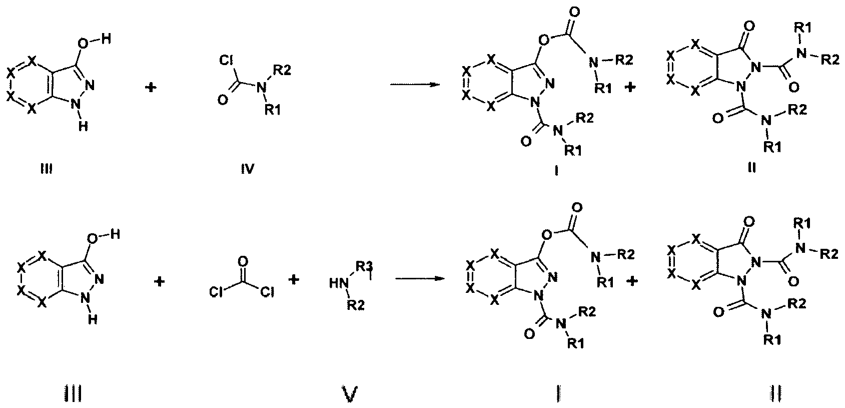

19. A process for preparing compounds of the formulae I and/or II with R2 hydrogen as claimed in claims 1 to 4, which comprises reacting indazole derivatives of the formula III with isocyanates of the formula VI: O=C=N-R1, and subsequently separating the compounds I and II by known processes.

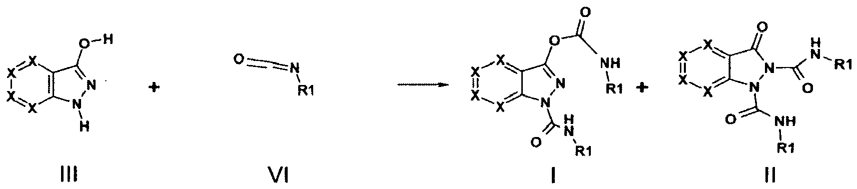

## Revendications

1. Composé de formule générale I ou II 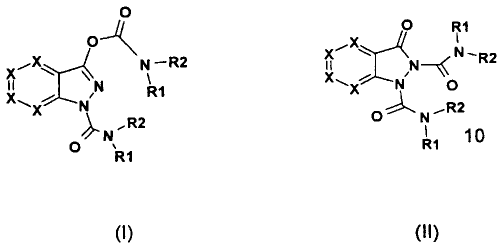 dans lesquelles les significations sont :
X de manière identique ou différente =C(-R)- ou =N-, où pas plus d'un X est =N- ;
R de manière identique ou différente hydrogène, halogène, hydroxy, (C₁-C₆)-alkyle, trifluorométhyle, (C₁-C₃)-alkyloxy ou amino ;
R1 de manière identique ou différente (C₆-C₁₀)-alkyle ou Y-phényle, qui peut être substitué par halogène, (C₁-C₆) -alkyle, (C₁-C₃) -alkyloxy ou trifluorométhyle ;
Y CH₂ ;
R2 hydrogène ; ou
R1 et R2 ensemble avec l'atome d'azote les portant forment un système de cycle monocyclique à 5 à 6 chaînons saturé, dont les chaînons individuels peuvent être remplacés par 1 à 3 atomes ou groupes atomiques de la série -CHR4-, -NR4-, -O-, -S-, à condition que deux motifs de la série -O-, -S- , ne puissent pas être adjacents ;
R4 (C₁-C₆)-alkyle, trifluorométhyle ou phényle,
les formes tautomères des composés et les sels physiologiquement tolérés de ceux-ci.

2. Composé de formule I ou II selon la revendication 1, **caractérisé en ce que**
NR1R2 est un système de cycle monocyclique à 5 à 6 chaînons saturé qui contient en position 4 un atome ou un élément atomique de la série -CHR4-, -CR4R5-, -(C=R4)-, -NR4-, -O-, -S-.

3. Composé de formule I ou II selon la revendication 1, **caractérisé en ce que**
X est de manière identique ou différente =C(-R)- ou =N-, où pas plus d'un X est =N- ;
R est de manière identique ou différente hydrogène, fluor, chlore, hydroxy, méthyle, trifluorométhyle ou amino ;
R1 est de manière identique ou différente (C₆-C₁₀)-alkyle ou Y-phényle, qui peut être substitué par méthyle ;
Y CH₂ ;
R2 hydrogène ; ou
R1 et R2 ensemble avec l'atome d'azote les portant forment un système de cycle monocyclique à 5 à 6 chaînons saturé, dont les chaînons individuels peuvent être remplacés par 1 à 3 atomes ou groupes atomiques de la série -CHR4-, -NR4-, -O-, -S-, à condition que deux motifs de la série -O-, -S- ne puissent pas être adjacents ;
R4 est méthyle, trifluorométhyle ou phényle, qui peut être substitué une ou deux fois par méthyle ou chlore ;
les formes tautomères des composés et les sels physiologiquement tolérés de ceux-ci.

4. Composé de formule I ou II selon la revendication 1, 2 ou 3, **caractérisé en ce que**
NR1R2 est pipéridine, qui contient l'élément atomique -CHR4- en position 4.

5. Composé de formule I ou II selon les revendications 1 à 4, **caractérisé en ce que**
X est de manière identique ou différente =C(-R)-.

6. Composé de formule I ou II selon les revendications 1 à 4, **caractérisé en ce que**
X en position 4, 5 et 6 est de manière identique ou différente =C(-R)-, et en position 7 est =N-.

7. Composé de formule I ou II selon les revendications 1 à 5, **caractérisé en ce que**
X en position 4, 5 et 7 est =C(-R)- avec R = hydrogène, et en position 6 R n'est pas hydrogène.

8. Médicament comprenant un ou plusieurs composés de formule I ou II selon les revendications 1 à 7.

9. Composé de formule I ou II selon les revendications 1 à 7 pour le traitement et/ou la prévention de troubles du métabolisme des acides gras et de troubles de l'utilisation du glucose.

10. Composé de formule I ou II selon les revendications 1 à 7 pour le traitement et/ou la prévention de troubles dans lesquels la résistance à l'insuline est impliquée.

11. Composé de formule I ou II selon les revendications 1 à 7 pour le traitement et/ou la prévention du diabète sucré et des séquelles associées à celui-ci.

12. Composé de formule I ou II selon les revendications 1 à 7 pour le traitement et/ou la prévention des dyslipidémies et de leurs séquelles.

13. Composé de formule I ou II selon les revendications 1 à 7 pour le traitement et/ou la prévention d'affections associées au syndrome métabolique.

14. Composé de formule I ou II selon les revendications 1 à 7 pour le traitement et/ou la prévention d'affections associées à un taux d'HDL réduit.

15. Composé de formule I ou II selon les revendications 1 à 7 pour le traitement et/ou la prévention de troubles athérosclérotiques.

16. Composé de formule I ou II selon les revendications 1 à 7 en combinaison avec au moins un autre principe actif pour le traitement et/ou la prévention de troubles dans lesquels la résistance à l'insuline est impliquée.

17. Procédé de préparation d'un médicament comprenant un ou plusieurs des composés de formule I ou II selon les revendications 1 à 7, **caractérisé en ce que** ces derniers sont mélangés avec un support pharmaceutiquement acceptable et ce mélange est transformé en une forme adaptée à l'administration.

18. Procédé de préparation de composés des formules générales I et/ou II selon les revendications 1 à 7, **caractérisé en ce que** des dérivés d'indazole de formule III
a) sont acylés avec des chlorures de carbamoyle de formule IV ;
ou
b) sont mis en réaction en deux étapes initialement avec du phosgène ou des équivalents tels que le chlorocarbonate de trichlorométhyle, le carbonate de ditrichlorométhyle ou le chloroformiate de 4-nitrophényle et dans une deuxième étape avec une amine V,
dans lesquels les substituants ont les significations mentionnées ci-dessus,
et par la suite la séparation des composés I et II est effectuée par des procédés connus.

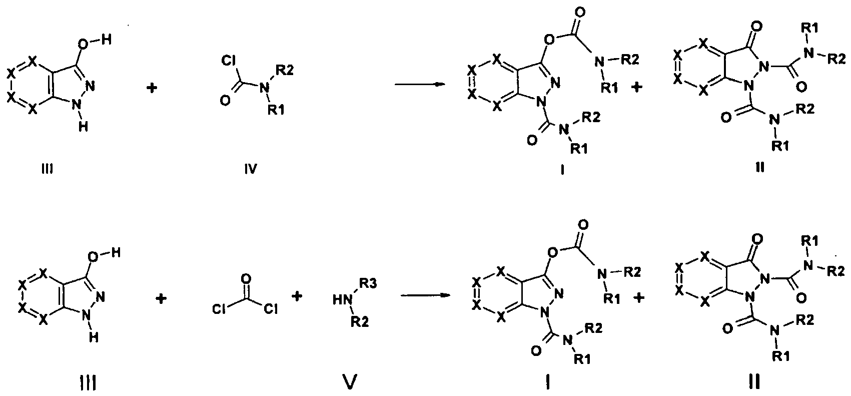

19. Procédé de préparation de composés des formules générales I et/ou II avec R2 hydrogène selon les revendications 1 à 4, **caractérisé en ce que** des dérivés d'indazole de formule III sont mis en réaction avec des isocyanates de formule VI : O=C=N-R1, et par la suite la séparation des composés I et II est effectuée par des procédés connus.
